(19) Europäisches Patentamt
European Patent Office
Office européen des brevets

(11) EP 0 783 326 B1

(12) EUROPEAN PATENT SPECIFICATION

(45) Date of publication and mention of the grant of the patent:
**04.02.2004 Bulletin 2004/06**

(21) Application number: **94929190.0**

(22) Date of filing: **07.09.1994**

(51) Int Cl.[7]: **A61K 51/10**, C07K 16/46

(86) International application number:
**PCT/US1994/010304**

(87) International publication number:
**WO 1996/007435 (14.03.1996 Gazette 1996/12)**

(54) **PROCESS FOR PREPARING MACROCYCLIC CHELATING AGENTS AND FORMATION OF CHELATES AND CONJUGATES THEREOF**

VERFAHREN ZUR HERSTELLUNG VON MAKROZYKLISCHEN AGENTIEN UND IHREN CHELATEN UND KONJUGATEN

PROCEDE DE PREPARATION D'AGENTS CHELATEURS MACROCYCLIQUES ET FORMATION DE CHELATES ET LEURS CONJUGUES

(84) Designated Contracting States:
**AT BE CH DE DK ES FR GB GR IE IT LI LU MC NL PT SE**

(43) Date of publication of application:
**16.07.1997 Bulletin 1997/29**

(73) Proprietor: **THE DOW CHEMICAL COMPANY**
**Midland, Michigan 48674 (US)**

(72) Inventor: **CHENG, Roberta, Chi-Lin**
**Midland, MI 48642 (US)**

(74) Representative:
**Weiss, Wolfgang, Dipl.-Chem. Dr. et al**
**Weickmann & Weickmann**
**Patentanwälte**
**Kopernikusstrasse 9**
**81679 München (DE)**

(56) References cited:
**EP-A- 0 353 450**
**WO-A-93/20852**
**US-A- 5 006 643**

- **PURE & APPLIED CHEMISTRY, vol. 61,no. 9, 1989 pages 1637-1641, DAVID PARKER ET AL. 'IMPLEMENTATION OF AMCROCYCLE CONJUGATED ANTIBODIES FOR TUMOUR-TARGETING'**
- **ANAL. BIOCHEM. (1984), 142(1), 68-78 CODEN: ANBCA2;ISSN: 0003-2697, 1984 MEARES, CLAUDE F. ET AL 'Conjugation of antibodies with bifunctional chelating agents: isothiocyanate and bromoacetamide reagents, methods of analysis, and subsequent addition of metal ions'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 783 326 B1

## Description

The present invention relates to a process for preparing isothiocyanato functionalized macrocyclic chelating agents and to a process for conjugating the macrocyclic chelating agents to biological molecules.

Metal ions may be attached to biological molecules by means of bifunctional chelating agents. Such bifunctional chelating agents are compounds which contain a metal-binding moiety which forms a chelate with metal ions and a second functional group, which is chemically reactive in nature and is capable of forming a covalent bond with biological molecules. The reactive functionality is usually one of the various known useful chemically reactive groups such as bromoacetyl group, a diazonium ion, an isothiocyanate or a carboxylic acid derivative, the reactive functionalities are capable of binding to a protein (e.g., the lysine moiety of an antibody). The biological molecules usually recognize distinctive external or internal cell markers and, thus, act as target directing groups for the metal ion.

When the bifunctional chelating agent is covalently attached to an antibody having specificity for cancer or tumor cell epitopes or antigens, radionuclide chelates of such antibody/chelating agent conjugates are useful in diagnostic and/or therapeutic applications as a means of conveying the radionuclide to a cancer or tumor cell. See, for example, Meares et al., *Anal. Biochem.,* 142, 68-78 (1984); and Krejcarek et al., *Biochem. and Biophys. Res. Comm*., 77, 581-585 (1977).

Isothiocyanato functionalized derivatives of ethylenediaminetetraacetic acid (EDTA) and diethylenetriaminepentaacetic acid (DTPA) are reported in the literature and are used to link radioactive isotopes to antibodies. For example, see Gansow et al., *Inorg. Chem*., 25, 2772-2781 (1986); Meares et al., *Anal. Biochem.,* 142, 68-78 (1984); and U.S. Patent 4,454,106.

When using short-lived radionuclides, radionuclides with a high specific activity, radionuclides with high energy or a radionuclide with a combination of these properties, it is desirable to chelate the radionuclide to the target directing group as close as possible to the time of injection into a patient to provide maximum specific radioactivity and minimum degradation of the radioimmunoglobulin. When using chelating agents such as EDTA and DTPA, the rapid sequestration of metal ions by these chelating agents allows the preparation of the radionuclide/antibody/chelating agent conjugates to be prepared by activating the chelating agent, reacting the chelating agent with an antibody and then chelating the radionuclide to form a chelate conjugate followed by purification of the chelate conjugate.

However, although chelating agents such as EDTA and DTPA rapidly chelate the radionuclide, they suffer from the disadvantage that such binding is kinetically labile. In addition, the use of labile radionuclides for antibody labeling in this manner allows substitutionally labile trace metals (which may not be radioactive) to be incorporated into this chelate. Competition for such non-radioactive trace metals diminishes the biological efficacy of a radionuclide/antibody/chelating agent complex since a lower quantity of radionuclide is delivered to the target site.

Another disadvantage associated with chelating agents such as EDTA and DTPA is the premature release of the chelated radioactive isotope. To lower the rate of metal release *in vivo*, bifunctional chelating agents, based on macrocyclic ligands, such as DOTA (1,4,7,10-tetraazacyclododecane-N,N',N",N'"-tetraacetic acid), have been used. See, for example, Mirzadeh et al., *Bioconjugate Chem*., 1, 59-65 (1990); and Deshpande et al., *J. Nucl. Med*., 31, 473-479 (1990).

A disadvantage in the use of macrocylic chelating agents is the relatively slow chelation rates which take place at room temperature, see for example, Mirzadeh et al., *Bioconjugate Chem.,* 1, 59-65 (1990). To reduce the radiolysis which occurs with prolonged chelation times, the medium in which the chelation takes place is often heated to increase the rate of chelation. As the isothiocyanate functionality is heat sensitive, U.S. Patent 5,006,643 discloses the preparation of isocyanato functionalized macrocycles containing primary or secondary amines by first reacting the chelating agent with rhodium and then activating the chelate. Chelating gadolinium to a tetraazamacrocycle containing tertiary amines prior to activating with an isothiocyanate functional group is disclosed in U.S. Patent 4,885,363. Based on macrocycles of similar functionalities EP Application EP-A-0353450 discloses the chelation of samarium followed by activation of the chelate. Using the above procedures for preparation of metal ion/chelating agent/protein conjugates to be used in radioimmunotherapy or radiodiagnostics requires the formation of the chelate, activation of the chelate and then conjugation of the activated chelate to a protein in a short period of time to avoid a substantial loss in activity.

To avoid some of the difficulties in preparing conjugates containing macrocyclic chelating agents, PCT application WO 93/20852 published on October 28, 1993 discloses an improved process for preparing conjugates by purifying the macrocyclic chelating agents on acid washed silica gel. Purifying the chelating agents in this manner allows activation of the chelating agent with thiophosgene to form an isothiocyanate derivative prior to the chelation of a metal ion. The chelation of a metal ion in the disclosed process can proceed rapidly at room temperature. However, the disclosed process still suffers from the disadvantage that the metal ion is reacted with the chelating agent prior to conjugation with a biological molecule. This is particularly disadvantageous when the metal ion is a short-lived radionuclide.

It would therefore be advantageous to have a process which allows rapid conjugation of the chelating agent with a protein prior to chelation of the metal ion. Such a process would be particularly advantageous for reducing

radiolysis and protein aggregate formation when the metal ion is a radionuclide. Also, having a process wherein the yield and purity from each step is increased would be particularly advantageous in preparing compounds and compositions which will be used in pharmaceutical applications where the pharmaceutical composition must meet the purity criteria set forth by various regulatory agencies.

The present invention is defined by the subject matter of claims 1 - 21. Particularly, the present invention is to a process for preparing chelate conjugates comprising:

(i) purifying a polyaza chelating agent by chromatography on a silica gel column wherein the silica gel has been acid washed prior to loading the polyaza chelating agent onto the column and the polyaza chelating agent is of the formula:

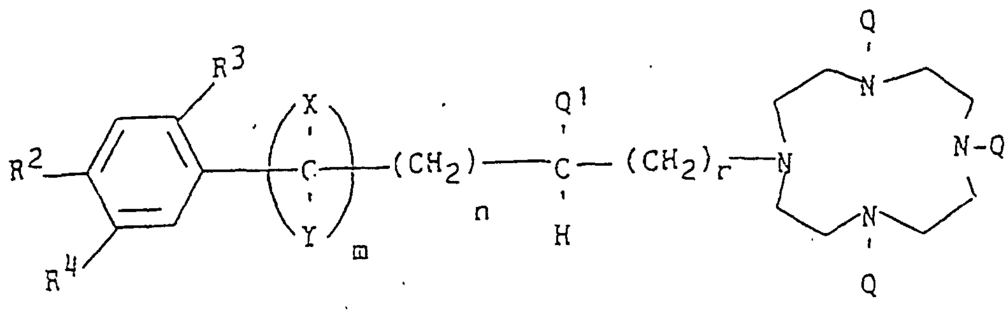

**(I)**

wherein:

each Q is independently hydrogen, $(CHR^5)_pCO_2R$; $(CHR^5)_pPO_3H_2$ or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen; and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$-$C_4$ alkyl;
X and Y are each independently hydrogen or may be taken with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is only 1 when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, $-OCH_2CO_2H$, hydroxy and hydrogen;

with the proviso that $R^2$ and $R^4$ cannot both be hydrogen but one of $R^2$ and $R^4$ must be hydrogen;

(ii) contacting the purified polyaza chelating agent with thiophosgene in an aqueous environment in the absence of an organic solvent at a pH from about 1 to about 5 to form an iosthiocyanato activated polyaza chelating agent and recovering the isothiocyanato activated polyaza chelating agent;

(iii) contacting the isothiocyanato activated polyaza chelating agent with a protein to form a chelating agent conjugate and recovering the chelating agent conjugate;
and
(iv) contacting the chelating agent conjugate with a radionuclide to form a chelate conjugate and recovering the chelate conjugate.

The present invention also provides a process for preparing chelate conjugates comprising:

(i) purifying a polyaza chelating agent, as defined in Formula I;
(ii) contacting the purified polyaza chelating agent with thiophosgene to form an isothiocyanato activated polyaza chelating agent and recovering the isothiocyanato activated polyaza chelating agent;
(iii) contacting the isothiocyanato activated polyaza chelating agent with a biological molecule to form a chelating agent conjugate;
(iv) contacting the chelating agent conjugate with a metal ion to form a chelate conjugate and recovering the chelate conjugate;

the improvement which comprises:

in step (i): purifying the polyaza chelating agent by chromatography on a silica gel column, wherein the silica gel has been acid washed prior to loading the polyaza chelating agent onto the column;
in step (ii): contacting the purified polyaza chelating agent with thiophosgene in an aqueous environment in the absence of an organic solvent at a pH from 1 to 5; and
in step (iii): contacting the isothiocyanato activated polyaza chelating agent with a biological molecule at between 25°C to 40°C.

The present invention is also to a chelate conjugate prepared by any of the aforementioned processes. The present invention also provides for a chelating agent conjugate prepared by the process of:

(i) purifying a polyaza chelating agent by chromatography on a silica gel column, wherein the silica gel has been acid washed prior to loading the polyaza chelating agent onto the column and the polyaza chelating agent is of the formula:

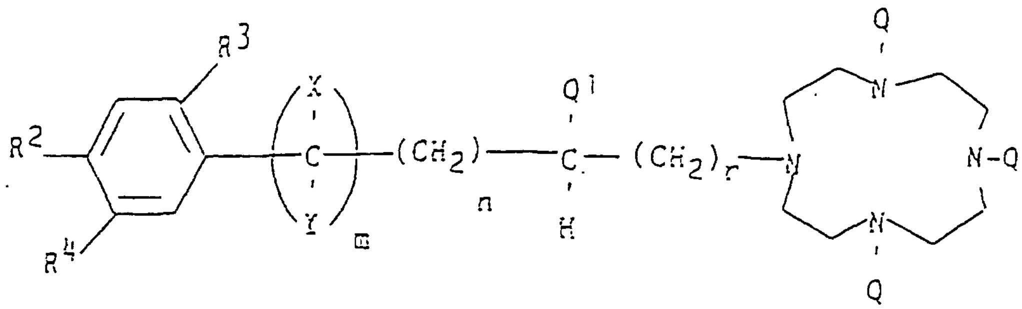

**(I)**

wherein:

each Q is independently hydrogen, $(CHR^5)_pCO_2R$; $(CHR^5)_pPO_3H_2$ or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen; and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$-$C_4$ alkyl;
X and Y are each independently hydrogen or may be taken with an adjacent X and Y to form an additional carbon-carbon bond;

n is 0 or 1;
m is an integer from 0 to 10 inclusive;
p = 1 or 2;
r = 0 or 1;
w = 0 or 1;

with the proviso that n is only 1 when X and/or Y form an additional carbon-carbon bond and the sum of r and w is 0 or 1;

$R^2$ and $R^4$ are selected from the group consisting of hydrogen and amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
(ii) contacting the purified polyaza chelating agent with thiophosgene to form an isothiocyanato activated polyaza chelating agent and recovering the isothiocyanato activated polyaza chelating agent; and
(iii) contacting the isothiocyanato activated polyaza chelating agent with a biological molecule to form a chelating agent conjugate.

The ability to prepare a chelating agent conjugate prior to addition of a metal ion to form a chelate conjugate has several advantages, particularly when the metal ion is a radionuclide and the chelate conjugate is used as a radiopharmaceutical or radiodiagnostic agent. These advantages include: a) the chelating agent conjugate can be prepared efficiently, is substantially metal free and can be used for application at any time with adequate storage; b) the process is simplified and radiation hazards are greatly reduced by the fact that radioactive material is not involved in most of the preparation; c) radiolabeling of the chelating agent conjugate requires only a few minutes of contact time with the radioactive material and damage to the biological molecule due to radiolysis is minimized; and d) radiolabeling and purification steps are simplified and could be performed by a radiopharmacist at the site of application with little need for sophisticated instrumentation. The latter process could also be performed with simple liquid transferring devices which would aid in limiting the exposure to the radioactive material.

In another embodiment, the present invention is to a chelating agent conjugate comprising a biological molecule conjugated to a chelating agent of Formula I wherein the chelating agent conjugate contains less than about three percent aggregate formation as measured by high performance liquid chromatography.

In still another embodiment, the present invention is to a chelate conjugate comprising a metal ion, a chelating agent and a biological molecule wherein the metal ion is bound by the chelating agent, the chelating agent is conjugated to the biological molecule and the chelating agent is of Formula I wherein the chelate conjugate contains less than about five percent aggregate formation as measured by high performance liquid chromatography.

As used herein, the term "chelating agent" means a compound capable of chelating or sequestering a metal ion. The term "chelate" means a chelating agent which has chelated a metal ion.

The term "bifunctional chelating agent" refers to compounds that have a moiety capable of chelating a metal ion and a linker/spacer moiety covalently bonded to the chelating moiety that is capable of being activated or functionalized to serve as a means to covalently attach to a biological molecule.

The term "biological molecule" refers to any hormone, antigen, hapten or protein, such as an antibody or antibody fragment, which functions to recognize a specific biological target site. Such a biological molecule, when attached to a functionalized chelate, serves to carry the attached metal ion to specific targeted tissues. Preferably, the biological material is a protein. More preferably the biological material is an antibody or antibody fragment.

As used herein, "antibody" refers to any polyclonal, monoclonal, chimeric, heteroantibody, recombinant or derivatives thereof. Preferably the antibody is a monoclonal antibody. As used herein the term "antibody fragment" includes Fab fragments and $F(ab')_2$ fragments and any portion of an antibody, including recombinants and derivatives thereof, having specificity toward a desired epitope or epitopes. The antibody fragments may be produced by conventional enzymatic methods or by genetic or protein engineering techniques, such as the production of single chain antibodies.

The terms "activated" or "activating" in relation to a chelating agent means the chelating agent has been modified with a functional group which is capable of forming a covalent bond with a biological molecule.

The term "chelating agent conjugate" as used herein refers to a biological material covalently attached to a bifunctional chelating agent. The term "chelate conjugate" as used herein refers to a biological material covalently attached to a chelate. The term "antibody/chelate conjugate" refers to an antibody which is covalently attached to a bifunctional chelate, (i.e., the bifunctional chelating agent having a chelated metal ion). The term "extraneous metal ion" as used herein means any metal ion other than a desired metal ion intentionally placed in contact with the chelating agent to form a chelate.

A general overall procedure giving the steps for preparation of conjugates used in the present invention is given in the following Scheme I.

**Scheme I**

Synthesis of a Chelating Agent

↓

Purification of the Chelating Agent on Silica Gel

Step (a)

↓

Activation of the Chelating Agent

Step (b)

↓

Conjugation of the Chelating Agent to a Biological Molecule to Form a Chelating Agent Conjuate

Step (c)

↓

Chelation of a Metal Ion by the Chelating Agent Conjugate to Form a Chelate Conjugate

Step (d)

↓

Purification of the Conjugate

The ability to form a chelating agent conjugate prior to the chelation of a metal ion, as shown in Scheme I, has several advantages, as stated above, particularly when the metal ion is a radionuclide. A particular advantage is

the chelating agent conjugate can be prepared, stored and the metal ion chelated shortly before use.

The formation of a chelating agent conjugate by the process of the present invention results in a chelating agent conjugate that generally contains less than about three percent aggregate formation as measured by high performance liquid chromatography (HPLC). Preferably the chelating agent conjugate formed by a process of the present invention contains less than about two percent aggregate formation as measured by HPLC. More preferred, the chelating agent conjugates prepared by a process of the present invention contains less than about one percent aggregate formation.

A chelate conjugate prepared by the process of the present invention generally contains less than about five percent aggregate formation as measured by HPLC. Preferably the chelate conjugate has less than about four percent aggregate formation. More preferably the chelate conjugate has less than three percent aggregate formation.

A chelate conjugate prepared by the process of the present invention also allows incorporation of the desired metal ion into a high percentage of chelating agent conjugates.

It has been unexpectedly found that when preparing chelate conjugates by the present invention, more chelating moieties can be added per antibody molecule, e.g., greater than two, without affecting the immunoreactivity of an antibody. It has also been unexpectedly found that after storage (e.g. - 70°C) of a chelating agent conjugate prepared by the process of the present invention, the chelate conjugates subsequently prepared have a higher immunoreactivity, less aggregate formation, and in animal studies show greater uptake in tumor tissue with less uptake in normal tissue than material prepared by a comparative process where the metal ion is chelated prior to forming the chelate conjugate.

Acid washing of the silica gel prior to use of the gel for purifying a chelating agent provides a process for reducing extraneous metal ions, specifically calcium ions ($Ca^{2+}$), during the purification of macrocylic chelating agents by flash chromatography using silica gel as disclosed in PCT application WO93/20852 published on October 28, 1993. The washing of the silica gel with a strong mineral acid, such as hydrochloric, nitric, sulfuric, perchloric or hydrobromic, prior to use substantially reduces the amount of undesired metal ions bound to the chelating agent during the purification process. Preferably, the mineral acid used to wash the silica gel is hydrochloric acid. Procedures for washing silica gel with a strong acid to remove impurities are known in the art, see, for example, Ralph K. Iler, *The Chemistry of Silica*, John Wiley & Sons (1979).

The elimination of undesired metal ions from the macrocyclic chelating agents during the initial purification results in several advantages in the subsequent steps for forming a chelate conjugate. The elimination of undesired bound metal ions from the macrocyclic chelating agents allows rapid chelation of the desired metal ions at room temperature as opposed to heating or long reaction times required to displace the calcium. The ability to perform the chelation step at room temperature, rather than at temperatures which would destroy the functionalizing group, allows the activation of the macrocyclic bifunctional chelating agent prior to chelation of the metal ion. The ability to functionalize the macrocyclic chelating agent prior to chelation of the metal ion is particularly advantageous as the activated chelating agent can be synthesized, conjugated to a biological molecule to form a chelating agent conjugate, stored if desired, and then reacted with the metal ion shortly before use. This is particularly advantageous when the metal ion is a radionuclide with a half life of 10 days or less, as a significant amount of radiolysis can occur during the time necessary for functionalizing the chelate, conjugation, and purification of the products from each step.

The present process can be used for many classes of ligands including any tri to hexa -(carboxamidomethylated), -(phosphonomethylated), or - (phosphinomethylated) polyazamacrocycle where the polyazamacrocycle has 3 to 6 nitrogens in the ring and the total number of atoms in the ring is 9 to 24 atoms. Of particular interest are aminocarboxylic acid chelating agents, aminophosphonic chelating agents and polyaza chelating agents containing secondary and tertiary amines. Preferred polyazamacrocyclic chelating agents are of the formula:

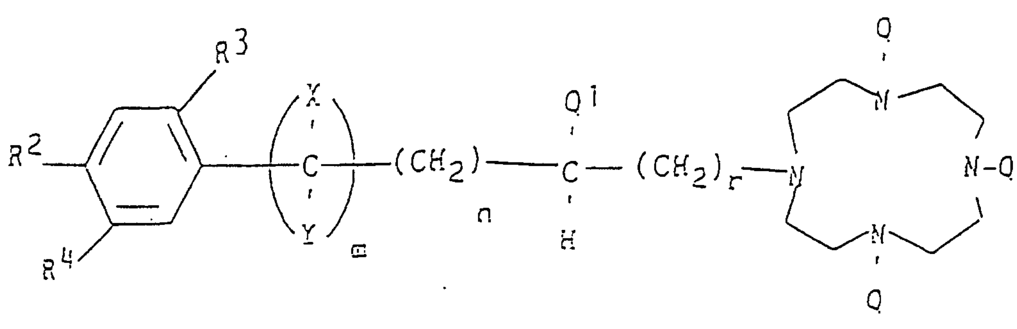

**(I)**

wherein:

each Q is independently hydrogen, $(CHR^5)_pCO_2R$,$(CHR^5)_pPO_3H_2$ or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen; and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$-$C_4$ alkyl;
X and Y are each independently hydrogen or may be taken with an adjacent X and Y to form an additional carbon-carbon bond;
n is 0 or 1;
m is an integer from 0 to 10 inclusive;
p = or 2;
r = 0 or 1;
w = 0 or 1;

with the proviso that n is only 1 when X and/or Y form an additional carbon-carbon bond and the sum of r and w is 0 or 1;

$R^2$ and $R^4$ are independently hydrogen or amino;
$R^3$ is $C_1$-$C_4$ alkoxy, $-OCH_2CO_2H$, hydroxy or hydrogen;

with the proviso that $R^2$ and $R^4$ cannot both be hydrogen but one of $R^2$ and $R^4$ must be hydrogen.

Preferred amines of the polyaza chelating agents of Formula I are tertiary amines, preferably where r is 0 and each Q is $(CHR^5)_pCO_2R$.

Procedures for synthesizing polyaza macrocycles are well known in the art. Examples of preferred chelating agents are given in Table I and are named as follows:

IA is 1-(4-aminophenyl)-1,4,7,10-tetra azacyclododecane- 1,4,7,10-tetraacetic acid;
IB is 1-[2-(4-aminophenyl)ethyl]-1,4,7,10-tetraazacyclododecane-4,7,10-triacetic acid;
IC is 1-[2-(4-aminophenyl)ethyl]-1,4,7,10-tetraazacyclododecane-1,4,7,10-tetraacetic acid;
ID is 1-(5-amino-2-methoxybenzyl)-1,4,7,10-tetraazacyclododecane -4,7,10-triacetic acid;
IE is 1-(5-amino-2-hydroxybenzyl)-1,4,7,10-tetraazacyclododecane-4, 7,10-triacetic acid; and
IF is 1-[2-(4-aminophenyl)ethyl]-1,4,7,10-tetraazacyclododecane-1--(R,S,-acetic-4,7,10-tris-R-methylacetic) acid, the preparation of which is given in European Patent publication No. 0,420,942, published April 10, 1991.

The preparation of IA-IF is given in European Patent publication No. 0,353,450, published February 7, 1990, and European Patent publication No. 0,420,942, published April 10, 1991.

## TABLE I

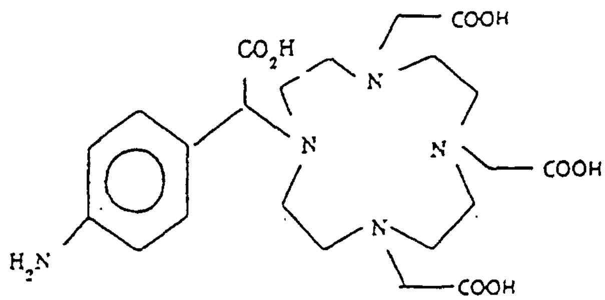

IA

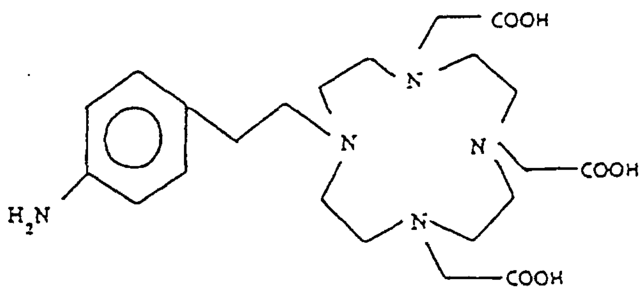

IB

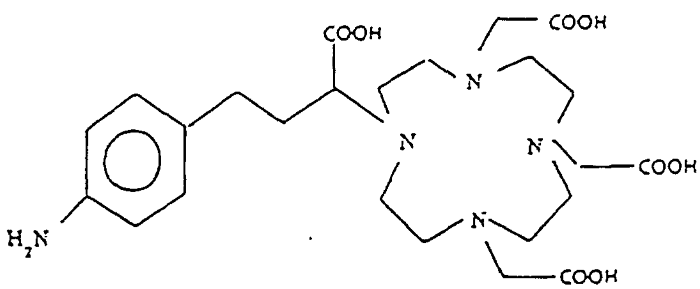

IC

TABLE I CONT'D

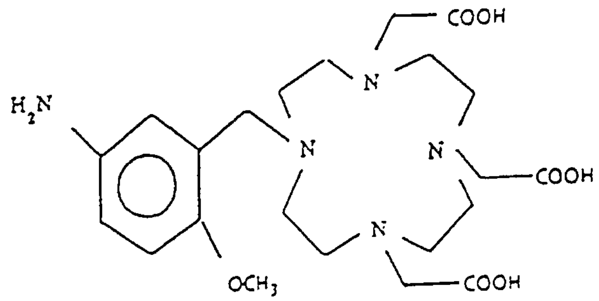

ID

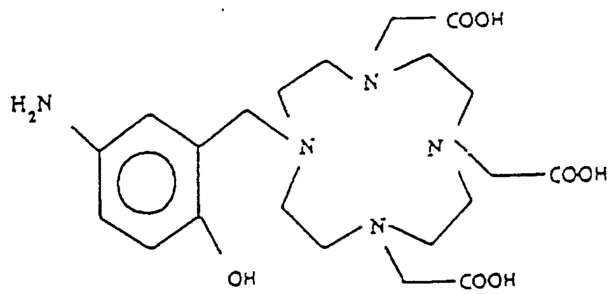

IE

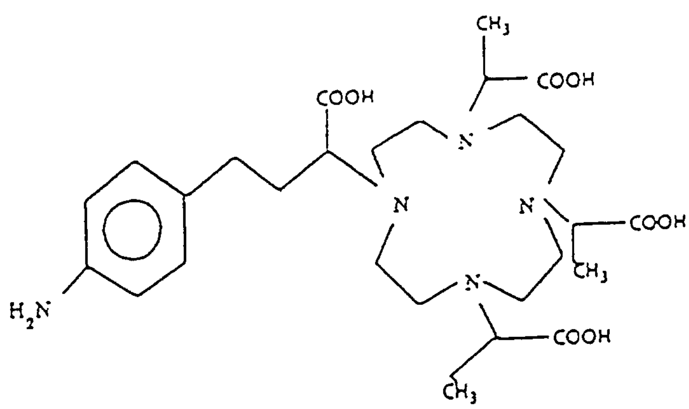

IF

Further the invention relates to a chelating agent conjugate comprising a protein conjugated to a chelating agent of the formula:

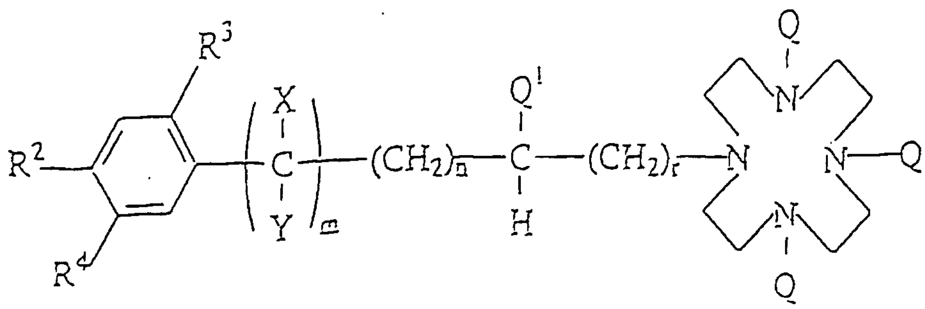

wherein:

each Q is independently hydrogen, $(CHR^5)_pCO_2R$, $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen;

each $R^5$ independently is hydrogen, $C_1$-$C_4$ alkyl, or -($C_1$-$C_2$ alkyl)phenyl;

with the proviso that at least one $R^5$ must be -($C_1$ - $C_2$ alkyl)phenyl;

each $R^6$ independently is -OH or $C_1$ - $C_4$ alkyl;
X and Y are each independently hydrogen or may be taken independently with an adjacent X or Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy,-$OCH_2CO_2H$,
hydroxy, and hydrogen;

with the proviso that $R^2$ and $R^4$ cannot both be hydrogen but one of $R^2$ and $R^4$ must be hydrogen;
wherein the chelating agent conjugate, upon conjugation of said chelating agent to said protein, contains less than about three percent aggregate formation as measured by high performance liquid chromatography.

Furthermore, the present invention is to a chelate conjugate comprising a radionuclide, a chelating agent and a protein wherein the radionuclide is bound by the chelating agent, the chelating agent is conjugated to the protein, and the chelating agent is of the formula as shown above, wherein the chelate conjugate, upon conjugation of said chelate to said protein, contains less than about three percent aggregate formation as measured by high performance liquid chromatography.

Methods for the carboxylation of an amine of a ligand to give amine derivatives containing a carboxyalkyl group are well known, as are the methods which give alkyl phosphonic, alkyl phosphinic and hydroxyalkyl substituents on the amine nitrogens. See, for example, U.S. Patents 3,726,912 and 3,398,198, Pulukkody et al. *Chem. Soc., Perkin Trans. 2,* page 605 (1993).

Aminophosphonic acid derivatives of ligands can be prepared by a number of known synthetic techniques. Of particular importance is the reaction of a compound containing at least one reactive amine hydrogen with carbonyl compound (aldehyde or ketone) and phosphorous acid or derivative thereof. [See the procedure of Moeoritzer and Irani, *J. Org. Chem.*, 31, 1603 (1966)].

The polyaza chelating agents of the present invention purified by flash chromatography on acid washed silica gel can be activated with any of the known functional groups capable of forming a covalent bond with a biological

molecule. Examples of such functional groups are isothiocyanate, bromoacetamide, maleimide, imidoester, thiophthalamide, diazonium and carboxyl. The use of polyaza chelating agents which are essentially free of undesired metal ions are particularly important when using functionalizing groups which are pH and/or thermally unstable, such as isothiocyanate. The activating functional groups are located at positions $R^2$ or $R^4$ of Formula I on the macrocyclic chelating agent.

The reaction of the thiophosgene with the chelating agent is at a pH from about 1 to about 7. Preferably, the pH is from 1 to 5. More preferably, the pH is from 1 to 3. The reaction is preferably carried out in an aqueous environment having an acid concentration of from about 0.005 to about 0.5 normal (N), preferably about 0.01 to about 0.2 N as disclosed in PCT application 93/20852 published on October 28, 1993. The acid can be any mineral acid, preferably hydrochloric acid. The reaction between the chelating agent and thiophosgene in a dilute acid with vigorous mixing and excess thiophosgene is very fast and usually complete in less than 5 minutes at room temperature (about 15°C to about 25°C). Higher or lower temperatures can be used (e.g., about 0°C to about 50°C) but room temperature is preferred.

The amount of excess thiophosgene added to the mixture depends on the concentration of the polyaza chelating agent. The lower the concentration of chelating agent, the larger the excess of thiophosgene to insure the rapid and complete conversion of amine to isothiocyanate. For example, if the concentration of chelating agent is $10^{-3}$ M, the ratio of thiophosgene to chelating agent is 5-20:1. If the concentration of chelating agent is $10^{-8}$ M, the ratio of thiophosgene to chelating agent is several thousand times larger (i.e., $10^5$:1). The excess thiophosgene is removed by conventional techniques such as evaporation, chromatography or extraction.

Rapid mixing of thiophosgene with the aqueous solution may be accomplished using conventional equipment known to those skilled in the art which is capable of producing sufficient shear to produce dispersion of the thiophosgene within the aqueous solution. Illustrative of such mixing means is the use of a Waring blender for large scale preparations and with a MIXXOR™ type mixer available from Alltech Inc., for smaller scale preparations.

By conducting the reaction of the chelating agent with thiophosgene as described above, the obtained purity of the isothiocyanato activated chelating agent is about 90 to about 95 percent as measured by high performance liquid chromatography.

The chelating agent can then be conjugated to a biological molecule. In a preferred embodiment, the biomolecule is a monoclonal antibody or fragment thereof which is specific for a selected cell-surface target site. Such antibodies may be commercially available or may be made by standard somatic cell hybridization techniques. An example of a suitable monoclonal antibody is CC49, one of a series of monoclonal antibodies specific for TAG-72 (tumor associated glycoprotein) described in published PCT Application No. WO 89/00692, on January 26, 1989, and published PCT Application No. WO 90/04410, on May 3, 1990. Generally, the chelate and protein are mixed in a molar ratio of greater than 1:10 and less than about 100:1 depending on the protein and protein concentration. Ratios of about 0.5: 1 to about 4:1 are preferred.

Methods for conjugating thiocyanate derivatized chelating agents to biological molecules are well known in the art. In general, when the biological molecule is a protein, an isothiocyanato linkage is formed with the ε-amino group of lysine. The conjugation generally involves reacting the functionalized chelating agent with the biological molecule from 2 to 18 hours in an aqueous buffered solution at pH 6-10 at room temperature.

The purity of the activated chelating agent obtained by the process of the present invention allows conjugation to a biological material, preferably a protein, to be done at temperatures from 25°C to 40°C, preferably 30°C to 40°C, without the expected increase in degradation of the chelating agent activated group (i.e., hydrolysis of the isothiocyanate) or protein. As the temperature of the conjugation reaction is increased, an increase in the rate of formation of protein aggregates and hydrolysis of the chelate activated groups would be expected in addition to an increased conjugation rate. Unexpectedly, elevated temperature results in a much faster conjugation rate increase relative to the rate of increase of undesired byproduct formation (e.g., chelating agent hydrolysis and protein aggregates).

The chelating agent conjugate can then be placed in contact with a metal ion to form a chelate conjugate. Although any metal ion, whether a radioactive metal ion or not, can be used, the chelate conjugate formed should have reasonable stability such that the metal complex is not readily disassociated. Radionuclides are preferred because of the use of the resulting products in a radiopharmaceutical drug for therapy and/or diagnosis. Preferred radioactive isotopes are those of samarium (Sm-153), holmium (Ho-166), ytterbium (Yb-175), lutetium (Lu-177), gadolinium (Gd-159), lanthanum (La-140), praseodymium (Pr-142), promethium (Pm-149), yttrium (Y-90) and indium (In-111).

Radionuclides can be produced in several ways. In a nuclear reactor a nuclide is bombarded with neutrons to obtain a radionuclide, e.g.,

$$\text{Sm-152} + \text{neutron} \rightarrow \text{Sm-153} + \text{gamma}$$

Another method of obtaining radionuclides is to bombard nuclides with particles produced by a linear accelerator or a cyclotron. Yet another way is to isolate the radionuclide from a mixture of fission products. The method of obtaining the nuclides employed in the present invention is not critical thereto.

A metal ion, such as a radionuclide can be complexed with the chelate conjugate by adding the chelate conjugates to a solution of the metal ion. Sequestration of the metal ion occurs readily upon mixing in an aqueous solution at a pH of 1 to 10. Preferably the reaction is carried out in a medium having a pH of 1 to 7 and more preferably 5 to 7. Ambient temperatures of 10°C to 40°C can be readily employed for metal ion chelation. The amount of metal ion employed may be from trace amounts to an amount in excess of equimolar with the chelate conjugate. Preferably, the sequestration of the metal ion occurs at room temperature, between 15°C to 22°C.

After formation of the chelate conjugate, an additional amount of a chelating agent can be added to remove any excess metal ion present.

When the sequestration of a metal ion by a chelating agent conjugate is done by the present process, it has been found that to facilitate chelation of the metal ion, the chelation should be done in a buffer selected such that the cationic moiety of the buffer does not associate with the chelating agent or that the anionic moiety of the buffer does not associate with a lanthanide metal ion. By "associate" is meant interference or inhibitions with the chelation of the metal ion by the chelating agent. For example, suitable buffers include alkali metal salts of acetate, and amine buffers which do not contain a primary amine. Examples of suitable amine buffers include N-[2-hydroxyethyl]piperazine-N'-[2-ethanesulfonic acid] (HEPES) and 2-[N-Morpholino]ethanesulfonic acid (MES). It has been found that standard buffers utilized for biological systems, such as phosphate, citrate, carbonate and succinate have a detrimental effect on chelation of metal ions by the chelating agent conjugates or chelate conjugates of the present invention, particularly when the metal ion is a lanthanide.

In the process of preparing chelate conjugates of the present invention, it is generally necessary to remove any buffer solutions present in the preparation of the chelating agent conjugate which may interfere with the subsequent step of chelating a metal ion. It has also been found that when preparing chelate conjugates by the present disclosed process, chelate conjugates can be purified more quickly and with greater efficiency than by the preparation of chelate conjugates wherein the metal ion is complexed with the chelating agent prior to forming a chelate conjugate. Purification of the chelate conjugates can be done utilizing standard techniques in the art, such as by gel filtration.

The invention will be further clarified by a consideration of the following examples, which are intended to be purely exemplary of the present invention.

In the following examples, the following terms are used.

BFC = bifunctional chelating agent.
DTPA = diethylenetriamine pentaacetic acid.
PA-DOTA = 1-[2-(4-aminophenyl)ethyl]-1,4,7,10-tetraazacyclododecane-1,4,7,10-tetraacetic acid.
HEPES = N-2-hydroxyethylpiperazine-N'-2-ethane sulfonic acid.
MES = 2-(morpholino)ethanesulfonic acid.

Mass spectra (fast atom bombardment with xenon) were obtained on a Vacuum Generators ZAB HS mass spectrometer. Samples for mass spectral analysis were prepared by dissolution in a 3:1 mixture of dithiothreitol:dithioerythritol (magic bullet) unless otherwise stated.

Analytical high performance liquid chromatography (HPLC) of non-radioactive samples was performed on a Hewlett Packard 1090 liquid chromatograph with a reverse phase column (4.6 x 100 mm Alltech Econosphere C18 3μ column) with a flow rate of 1 mL per minute and detection at 254nm. Samples were eluted with a gradient of 0.05 M, pH 6.0, sodium acetate/acetonitrile.

HPLC of radioactive samples was performed using a gel filtration column (DuPont ZORBAX™ GF-250 9.4 x 250 mm) with a flow rate of 1.5 mL per minute and equipped with radioactivity and ultraviolet light detectors. The mobile phase was 0.25 M, pH 6.0 sodium citrate/10 percent acetonitrile.

In general, containers for the conjugation reaction and for storage of the conjugate were soaked in 10% (v/v) nitric acid for 48 hours and rinsed thoroughly with deionized water prior to contacting the antibody solution. Buffer solutions were either made with extra pure reagents or decontaminated by passage through a chelating resin (CHELEX™ 100, BioRad) before contacting any unlabeled antibody and/or conjugate. Chelation experiments in general were followed by HPLC with UV and radioactivity dual detectors.

**Examples**

Example 1 Purification of PA-DOTA by Flash Chromatography

A 1.5 x 14 inch flash chromatography column was packed with 17.0 g of Merck 60 A silica gel, 230-400 mesh (Aldrich Chemical Co.) which had been acid-washed by Alltech Inc. Crude PA-$H_4$DOTA·3HCl (3.2g, 75 percent pure

by HPLC analysis) was applied to the column and eluted with a mobile phase of 2:2:1 chloroform:methanol:concentrated ammonium hydroxide. Fractions containing only PA-DOTA$(NH_4)_2$ as assayed by thin layer chromatography were collected and pooled to give PA-DOTA$(NH_4)_2$ which was greater than 98 percent area purity as assayed by HPLC analysis. The absence of calcium in the recovered PA-DOTA$(NH_4)_2$ was confirmed by mass spectrometry and HPLC using an elution buffer gradient of 95/5 sodium acetate (0.05M, pH = 6)/acetonitrile to 30/70 in 15 minutes.

Chelation of yttrium to the PA-DOTA obtained above was performed by dissolving 3 mg of PA-DOTA in 3 mL of 0.5 of sodium acetate buffer (pH = 6) and 0.2 mL of 40 millimolar $Y(OAC)_3 \cdot 4H_2O$, this represents approximately a 5 fold excess of yttrium. The extent of chelation was monitored by HPLC as described above.

Chelation with PA-DOTA which was purified using acid washed silica gel was complete in less than 5 minutes at room temperature (18-25°C).

Comparative Example A

Chelation with PA-DOTA purified using silica gel which has not been acid washed was 4 percent complete in 15 minutes at room temperature and was 86 percent complete in 10 minutes at 90°C.

These results show acid washing the silica gel to remove extraneous metal ions prior to use for purifying the chelating agent greatly enhances the rate at which the desired metal is sequestered at room temperature.

Example 2 Preparation of SCN-PA-$H_4$DOTA·2HCl·2$NH_4$Cl

To 100 mL of 0.01 M hydrochloric acid was added 250 mg of calcium free PA-$H_2(NH_4)$DOTA(0.448 millimoles, 2 of the carboxylated groups are protonated and 2 of the carboxylated groups are ammonium salts) as prepared in Example 1, and the solution placed in a 40-oz. Waring blender. Thiophosgene (170 microliters, 2.23 millimoles) was added and the blender quickly started. After 2 minutes of mixing, the mixture was added to a separatory funnel and excess thiophosgene was extracted with three 50 mL portions of chloroform. The aqueous layer was added to 100 mL of acetonitrile and the solution reduced to dryness on a rotary evaporator (fitted with a vacuum pump) at room temperature. The solid obtained was further dried for 2 hours at room temperature on a vacuum line. The yield was 321 mg (90 percent yield) of an off-white solid.

Characterization of the product by mass spectrometry and by HPLC showed the product was 96 percent by weight an isothiocynate derivatized PA-DOTA(SCN-PA-$H_4$DOTA·2HCl·2$NH_4$Cl). The HPLC elution gradient being sodium acetate/acetonitrile 95/5 to 70/30 in 15 minutes and then to 40/60 in 20 minutes.

Comparative Example B

Samples prepared in an identical manner except that the reaction was carried out by the addition of 20 to 50 percent by volume chloroform prior to mixing were 84 to 89 percent pure as measured by HPLC.

These results show that a high purity isothiocyanato activated chelating agent can be obtained by mixing the thiophosgene and chelating agent in an aqueous system in the absence of an organic solvent.

Example 3 Preparation of [$^{177}$Lu(SCN-PA-DOTA)]

To 20 µL of SCN-PA-DOTA (4.88 x $10^{-3}$ moles in water) prepared as described in Example 2 was added 100 µL of Lu-177 (50 microcurries, 1mM in 0.05N HCl) to give a molar ratio of chelating agent to Lu-177 of about 1:1. The solution was mixed on a vortex mixer for about 5 seconds, 100 µL of HEPES buffer (0.5 M, pH 7) were added and the solution mixed for 5 minutes. The pH was measured and adjusted to between pH 6-7 with HEPES buffer if necessary. The reaction was allowed to proceed for another 5 minutes at room temperature and the yield of [$^{177}$Lu(SCN-A-DOTA)] was 91.4 percent as determined by HPLC.

The [$^{177}$Lu(SCN-PA-DOTA)] complex was purified by placing the sample on a reverse phase cartridge (PRP-1 MINI-CLEAN™ column, 80 µL) which had been pretreated with 800 µL acetonitrile, 400 µL of water and 800 µL 10 percent by volume acetonitrile in 20 mM carbonate buffer, pH 9.5. After loading the complex onto the column, the reaction vial was rinsed with a 200 µL and then a 600 µL of 10 percent by volume acetonitrile in carbonate buffer, the washes also being placed on the column.

The complex was then eluted from the column using a 1:2 ratio of carbonate buffer (20 mM, pH 9.5):acetonitrile. Approximately 80 percent of the radioactivity was recovered in the second 50 µL elution volume.

Example 4 Conjugation of [177Lu(SCN-PA-DOTA)] With IgG CC49 at Room Temperature (21-22°C)

To 257 µL of an antibody solution (IgG CC49, 14.5 mg/mL in 50 mM carbonate buffer, pH 9.5) was added 25

μL of [177Lu(SCN-PA-DOTA)] prepared as described above, giving a BFC: antibody molar ratio of about 1.36. The activated chelate and antibody solution was mixed on a vortex mixer for about 10 seconds and allowed to stand at room temperature for approximately 2 hours with mixing about every 10 to 15 minutes. The disappearance of the [$^{177}$Lu (SCN-PA-DOTA)] was measured by HPLC. After 120 minutes, about 62 percent of the $^{177}$Lu activity was associated with the antibody, about 7 percent was non-antibody associated impurities, and unreacted [$^{177}$Lu(SCN-PA-DOTA)] was about 30 percent.

Example 5 Conjugation of [$^{177}$Lu(SCN-PA-DOTA)] With IgG CC49 at 37°C

To an antibody solution (15.7 mg CC49IgG (39 nanomoles) in 286 μL of carbonate buffer, 20 mM, pH 9.5) was added 21 nanomoles of [$^{177}$Lu(SCN-PA-DOTA)] complex (9.7 millicurries of activity) in 11 μL of the eluent from Example 3. The mixture was mixed on a vortex mixer and allowed to stand in a 37°C oven for one hour with mixing about every 10 to 15 minutes. The progress of the reaction was monitored by HPLC analysis for the disappearance of the complex as described in Example 4. After 1 hour the $^{177}$Lu activity associated with the antibody as measured by HPLC was 91 percent, less than 4 percent of the $^{177}$Lu activity was associated with impurities, and 5 percent was unreacted [$^{177}$Lu(SCN-PA-DOTA)].

The conjugation reaction was terminated by isolation and purification of the conjugate on a gel filtration column (PD-10 column, SEPHADEX™ G-25, medium, 9 mL) which had been equilibrated with phosphate buffered saline.

Homogeneity of the labeled antibody was examined by HPLC and by SDS PAGE electrophoresis (sodium dodecyl sulfate-polyacrylamide electrophoresis) coupled with autoradediography. Immunoreactivity was determined by IRMA and affinity column binding and was found to be comparable to that conjugated at 25°C. *In vivo* biodistribution (in rats) of the conjugate prepared at 37°C was not significantly different from that prepared at 25°C.

The results show that the chelate can be rapidly conjugated to an antibody at 37°C without affecting the immunoreactivity or biodistribution of the conjugate.

Example 6 Conjugation of CC49 with SCN-PA-DOTA and the subsequent chelation with $^{177}$Lu

To an antibody solution of IgG CC49 (1.25 mL of about a 10.4 mg/mL solution) was added 1 mL of a DTPA solution (0.25 M, pH 6) and the solution concentrated in a concentrator (30 K, CENTRICON™) by centrifugation on a DuPont Sorvall centrifuge (RT 6000B) for 45 minutes at 5500 rpm. The DTPA treated antibody solution was washed 2 times with 1.5 mL each of a carbonate buffer (20 mM; pH 9.5 ± 0.1), and further concentrated to yield 0.9 mL of an antibody solution of approximately 12 mg/ml of CC49.

To 500 μL (40 nmole) of the above antibody solution was mixed 120 μL (120 nmole) of SCN-PA-DOTA (1 mM solution in $H_2O$). The pH was adjusted to about 9.5 with a sodium carbonate solution (1.0M) and the solution was incubated at 37°C for 2 hours. Conjugation was terminated by removal from heat an purification of the chelating agent conjugate by gel filtration chromatography with a column (PD-10 column, 9 mL bed vol of Pharmacia SEPHADEX™ G-25 preconditioned with 3 times with 6 mL of MES buffer (20 mM, pH6). The column was eluted with the same MES buffer. The pass-through and the initial wash of 2.4 mL total volume were discarded. The next 1.5 to 2.0 mL of eluate, which contained the bulk of the chelating agent conjugate, were collected.

To the chelating agent conjugate that came off the PD-10 column was added 60 μL of $^{177}$Lu (1mM in 0.05 N HCl). This solution was mixed on a vortex mixer, and after 15 minutes at room temperature, 25 μL of a DTPA solution (0.25 M, pH 6) was added. The amount of $^{177}$Lu incorporation was determined by HPLC analysis. The $^{177}$Lu labeled CC49 isolated by gel filtration on a PD-10 column was shown to contain less than 1 % each of aggregates and non-bound $^{177}$Lu. Immunoreactivity of the labeled CC49 that contained 0.97 $^{177}$Lu-PA-DOTA molecules per antibody was found to be 96.0 ± 1.0% by affinity binding to BSM (mucin from bovine submaxillary glands).

Example 7 EDTMP as Scavenger for Removal of Unbound $^{177}$Lu from Radiolabeling Mixtures and the Subsequent Purification by Ion Exchange Chromatography.

A CC49 PA-DOTA conjugate was prepared and purified as described in Example 6 from 32 nmole (4.8 mg) of CC49 and 96 nmole of SCN-PA-DOTA in 600 μL of carbonate buffer at pH 9.5 for 3 hours. To the chelating agent conjugate in 2.0 mL of MES buffer (20 mM, pH 6.2) was added 64 μL of $^{177}$Lu(1 mM in 0.05 N HCl), followed after a few minutes by 20 μL of an ethylenediamine-tetramethylenephosphoric acid (EDTMP) solution (0.25 M, pH 6). HPLC analysis showed that 60 percent of $^{177}$Lu was antibody bound, and the remaining presumably bound to EDTMP. The solution was then divided into four equal portions, and each purified via one of the four columns below. Column 1 was a prepacked PD-10 from Pharmacia, Column 2 was packed with 3 mL of a strong anion exchange resin slurry (SEPHADEX™ QAE gel a diethyl[2-hydroxypropyl]-aminoethyl SEPHADEX™ G25; approximately 0.3 g of dry beads). Column 3 was a strong anion exchange prepacked column (BioRad AG 1 x 8,40 - 120 Mesh size), and Column 4 was a

quaternary amine on silica cartridge in a prepacked cartridge (SAX, from Varion). To each of the four columns, preconditioned with 3 x 5 mL of MES buffer, was loaded the reaction mixture and eluted with 4 mL of PBS (phosphate buffered saline; 120 mm NaCl, 2.7 mm KCl and 10 mm phosphate, pH7.4). Comparable purification, summarized in Table II, was achieved with any of these columns. The $^{177}$Lu labeled CC49 eluted off the anion exchange columns in 1-2 mL of PBS with good recovery (84-87%) and excellent homogenity of the purified labeled antibody.

TABLE II

| Purification of $^{177}$Lu CC49 by Gel Filtration or Anion Exchange Chromatography - EDTMP as the Chelating Agent | | | | |
|---|---|---|---|---|
| | GF PD-10 (Percent) | SEPHADEX™ QAE (Percent) | BioRad AGI x 8 (Percent) | Varion SAX (Percent) |
| $^{177}$Lu CC49 recovery, | 94 | 86 | 87 | 84 |
| non-protein associated $^{177}$Lu | 1.3 | 0.4 | 0.5 | 0.4 |
| $^{177}$Lu on aggregates | 1.2 | 0.9 | 0.4 | 0.9 |

Example 8 DTPA as Scavenger for Removal of Unbound $^{177}$Lu from Radiolabeling Mixtures and Subsequent Purification by Ion Exchange Chromatography

Monoclonal antibody CC49 was conjugated and radiolabeled as per Example 7, except that half of the conjugate was chelated at pH 7 in HEPES buffer, and DTPA (diethylenetriaminopentaacetic acid) was used to scavenge unbound Lu-177. The final purification was accomplished by ion exchange chromatography on either SEPHADEX™ QAE or DOWEX™ AG1 x 8 column pre-washed with 3 x 5 mL of deionized water. The labeled antibody was eluted off with PBS. Results shown in Table III are comparable to those described in Example 7.

TABLE III

| Purification of $^{177}$Lu CC49 by Anion Exchange Chromatography with DTPA as the scavenger | | | | |
|---|---|---|---|---|
| | SEPHADEX™ QAE | AG1 x8 | SEPHADEX™ QAE | AG1 x8 |
| pH of chelation (Buffer) | pH 6 (MES) | pH 6 (MES) | pH 7 (HEPES) | pH 7 (HEPES) |
| $^{177}$Lu CC49 recovery, | 86% | 87% | 86% | 87% |
| non-protein associated $^{177}$Lu | 0.4% | 0.5% | 0.4% | 0.5% |
| $^{177}$Lu on aggregates | 0.9% | 0.4% | 0.9% | 0.4% |

Example 9 Scale-up Preparation of MoAb CC49 PA-DOTA Conjugate

1. Conjugation:

Five hundred mL of antibody CC49 (8.8 mg/mL; 5.87 x $10^{-5}$ M/L) was preheated at 37°C in an acid washed flask. The pH of the antibody solution was adjusted to 9.5 ± 0.1 with a 1.0 M sodium carbonate solution (approximately 0.056 mL/mL of antibody in PBS). This was followed by the addition, with gently swirling, of 29.3 mL of an SCN-PA-DOTA solution (5 mM in sterile water for injection). The input molar ratio of SCN-PA-DOTA to antibody was 5:1. The solution was incubated in a water bath at 37°C for 2 hours. Upon termination of the conjugation reaction, the mixture was purified by gel filtration chromatography.

2. Purification of Antibody Conjugate:

A. Pretreatment of a Gel Column

To sanitize and to remove trace metal contaminants and substances that could possibly inhibit the subsequent chelation of the purified chelating agent conjugate, a gel column (Sephacryl S-200; Pharmacia BPG 200/950 column with bed volume = 20 L) was pretreated with a DTPA-sodium hydroxide solution (10 L of 50 mM DTPA in 0.7 M sodium hydroxide), followed by 100 L of sterile water for injection and 100 L of $NH_4OA_c$ buffer (25 mM; pH7.0).

B. In-process Test of Column Wash:

As a precautionary measure, an in-process functional test was implemented to assure that the column wash is free of DTPA or anything else that could adversely affect the chelation of the DOTA functionality. This test measures the chelation of PA-DOTA-SCN with $^{177}$Lu in the column effluent compared to pure solvent.

C. Gel Filtration Chromatography:

The monoclonal antibody chelating agent conjugate (PA-DOTA-CC49) mixture was loaded on the pre-treated gel filtration column (Sephacryl S-200, using a Pharmacia BIOPROCESS™ System I with a BPG 200/950 column). The purified monoclonal antibody chelating agent conjugate was eluted with an ammonium acetate buffer (25 mM, pH 7). The conjugate obtained had a protein concentration of approximately 1.5 to 2 mg/mL. It was further concentrated to around 5 - 6 mg/mL. The conjugate was then stored in aliquots at -70°C. In general a 80% over-all yield was achieved based on protein concentration.

3. Chelation of CC49 PA-DOTA Conjugate and Purification of the $^{177}$Lu labeled CC49

A PA-DOTA-CC49 conjugate prepared as above, (12.0 mL of 5.3 mg/mL of protein; 63.6 mg; 424 nanomole total), was thawed and allowed to warm to ambient temperature (approximately 21-22°C). To this was added 933 µL of a $^{177}$Lu solution (1 mM in 0.05 N HCl) and mixed for 15 seconds, the ratio of $^{177}$Lu to antibody was 2.2. After 15 minutes at room temperature, 460 µL of a DTPA solution was added (5 mM, pH 6.0) to the chelation mixture and mixed. A small sample of the reaction mixture was withdrawn and analyzed by HPLC for the amount of $^{177}$Lu bound to the antibody, which was found to be 93.0 percent. Purification of the $^{177}$Lu labeled CC49 was accomplished by passage of the reaction mixture through an anion exchange column (8g of SEPHADEX™ QAE). After discarding the pass-through, and 1 mL of rinse, the labeled antibody was eluted off in 30 mL of PBS containing 5 percent glycerol. A 89 percent yield, based on $^{177}$Lu activity, was obtained.

Example 10 Storage stability of CC49 PA-DOTA Conjugate

A PA-DOTA-CC49 conjugate was prepared as per Example 9 and stored in 0.9 mL aliquots in glass serum vials each sealed with a TEFLON™ coated gray rubber stopper and an aluminium cap at -70°C. One vial of the conjugate was removed and analyzed at certain time intervals for its chelatability with $^{177}$Lu (excess and limited amount) and retention of immunoreactivity by its binding to a BSM agrose gel. Results confirmed that the conjugate could be stored at -70°C for prolonged periods of time (assayed out to about 4 months) without significant loss of chelatability or immunoreactivity.

Example 11 Comparative Biodistribution of Chelate Conjugates Prepared by process A and Process B given in Scheme I.

To determine the effect of the procedure utilized in preparing chelate conjugates, chelate conjugates were prepared as outlined in Process A and B in Scheme I.

Sample 1 (Process A) was prepared by mixing equal molar quantities of SCN-PA-DOTA with $^{177}$Lu at pH 6-7,and purification by reverse phase chromatography. Conjugation of [$^{177}$Lu(SCN-PA-DOTA)]- with CC49 was done at pH 9.5 ± 0.1 for 90 min. at 37°C and yielded a chelate conjugate containing approximately 0.8 $^{177}$Lu/Ab.

Sample 2 (Process B), which contained 1.1 $^{177}$Lu/Ab, was prepared as described in Example 6. The samples were charaterized by HPLC immunoreactivity and trichloroacetic acid precipitation. Results, shown in Table IV, indicated that the two preparations were comparable with respect to the *in vitro* properties examined.

TABLE IV

| *In vitro* Tests of $^{177}$Lu CC49 for use in Biodistribution | | |
|---|---|---|
| Tests | Sample 1 (percent) | Sample 2 (percent) |
| HPLC: %$^{177}$Lu bound to aggregates | 1.2 | 0.5 |
| % $^{177}$Lu not associated with protein | 0.2 | 0.5 |
| Immunoreactivity by BSM, percent Bound | 90.8 ± 0.4 | 91.1 ± 0.2 |

TABLE IV (continued)

| *In vitro* Tests of $^{177}Lu$ CC49 for use in Biodistribution | | |
|---|---|---|
| Tests | Sample 1 (percent) | Sample 2 (percent) |
| TCA precipitation | <1 | 1 |

Biodistribution of studies of the chelate conjugates prepared above were performed in female Bulb/C mice 6-7 weeks old. The mice were dosed intravenously via tail vein injections at 5 hours, 24 hours, 2 days and 5 days, groups of 5 animals were euthanized and appropriate tissue samples removed and analyzed. Results shown in Table IVA for sample 1 and table IVB for sample 2 indicate there was no significant difference between the biodistribution of the two samples prepared by the two different routes.

TABLE IVA - Biodistribution of $^{177}$Lu-PA-DOTA CC49 Conjugate as Prepared by Process A

| Tissue | 5 Hours | | 24 Hours | | 48 Hours | | 120 Hours | |
|---|---|---|---|---|---|---|---|---|
| | Avg. | Std. | Avg. | Std. | Avg. | Std. | Avg. | Std. |
| Blood | 33.66 | 4.34 | 27.64 | 2.60 | 24.77 | 2.39 | 20.79 | 1.55 |
| Liver | 13.03 | 1.85 | 10.49 | 1.36 | 9.88 | 1.29 | 8.75 | 0.77 |
| Spleen | 10.81 | 2.07 | 11.40 | 1.59 | 10.65 | 1.16 | 10.42 | 0.62 |
| Kidney | 6.90 | 1.48 | 5.72 | 0.59 | 7.00 | 0.87 | 5.96 | 0.73 |
| Femur | 2.47 | 0.02 n=2,a | 3.89 | 0.51 | 3.65 | 0.31 | 3.43 | 0.33 |

a One tissue value omitted due to Outlier Statistic >p-95 value.
b One animal omitted, tail vein >10 percent injected dose.
c One animal omitted, tissues incorrectly weighed.

TABLE IVB - Biodistribution of $^{177}$Lu-PA-DOTA CC49 Conjugate as Prepared by Process B

| Tissue | 5 Hours | | 24 Hours | | 48 Hours | | 120 Hours | |
|---|---|---|---|---|---|---|---|---|
| | Avg. | Std. | Avg. | Std. | Avg. | Std. | Avg. | Std. |
| Blood | 35.24 | 4.15 | 27.51 | 0.35 n=4,a | 24.53 | 2.18 | 20.96 | 1.22 |
| Liver | 13.70 | 3.12 | 9.97 | 0.81 | 10.85 | 1.23 | 8.80 | 0.28 n=4,a |
| Spleen | 9.46 | 0.55 (n=4,a) | 9.57 | 1.06 | 10.83 | 0.80 | 10.09 | 0.67 |
| Kidney | 7.60 | 2.42 | 5.24 | 0.43 n=4,a | 5.67 | 0.85 | 6.69 | 0.65 |
| Femur | 3.48 | 0.81 | 3.59 | 0.29 | 3.75 | 0.42 | 3.51 | 0.23 |

a One tissue value omitted due to Outlier Statistic >p-95 value.
b One animal omitted, tail vein >10 percent injected dose.
c One animal omitted, tissues incorrectly weighed.

**Example 12** Biodistribution of $^{177}Lu$ CC49 chelate conjugates in Tumor Bearing Mice

To determine the effect of the number of radionuclides bound to an antibody molecule, as prepared by Process B, two samples were prepared containing approximately 1.2 or about 2 $^{177}Lu$/antibody molecule.

Two samples were prepared using antibody conjugate prepared and labeled with 177Lu as described in Example 9 with input ratios of BFCA/Ab = 3 (Sample 3) and 5 (Sample 4) for conjugation. *In vitro* integrity and immunoreactivity of the two samples were comparable as shown in Table V.

TABLE V

| *In vitro* **Tests** | | |
|---|---|---|
| Tests | Sample 3 | Sample 4 |
| Number of $^{177}Lu$/Ab | 1.2 | 2.0 |
| HPLC: %$^{177}Lu$ bound to aggregates | 1.4% | 1.0% |
| % $^{177}Lu$ not associated with protein | 0.6% | 0.5% |
| Immunoreactivity by BSM, % Bound | 97.0 ± 0.01% | 96.5 ± 0.78% |

A biodistribution study utilizing samples as prepared above was performed in female CD-1 athymic (nu/nu) mice of 6-7 weeks old bearing a LS-174 T human colon carcinoma xenografts. The mice were dosed intravenously via tail vein injections and at various time intervals, groups of 5 animals were euthanized and tissue samples removed and analyzed. The results given in Table VA and VB indicated that there is a slight difference in the tumor uptake and whole body retention, particularly at longer time points, i.e., at 120 hours. No significant difference between the two samples was observed in other tissues. The results also indicate that more than one chelate can be conjugated to an antibody molecule without adversely affecting the biodistribution of the labeled antibody.

TABLE VA:Biodistribution of 177Lu-PA-DOTA CC49 Conjugates having a Ratio of 1.2 BFC:Ab, n=5

| Tissue | 5 Hours | | 24 Hours | | 48 Hours | | 120 Hours | |
|---|---|---|---|---|---|---|---|---|
| | Avg. | Std. | Avg. | Std. | Avg. | Std. | Avg. | Std. |
| Blood, Total | 37.95 | 2.28 | 26.56 | 3.39 | 21.73 | 1.48 | 15.46 | 2.65 |
| Liver | 10.86 | 0.60 | 9.46 | 0.85 n=4,a | 8.76 | 0.55 n=4,a | 8.08 | 2.28 |
| Spleen | 0.85 | 0.12 | 0.72 | 0.16 | 0.72 | 0.17 | 0.66 | 0.23 |
| Kidney | 1.15 | 0.32 | 0.77 | 0.05 n=4,a | 0.95 | 0.16 | 0.86 | 0.08 n=4,a |
| Tumor | 3.20 | 1.17 | 10.73 | 3.04 | 11.10 | 3.22 n=4,c | 15.39 | 2.75 n-4,c |
| Femur | 0.17 | 0.02 | 0.14 | 0.01 | 0.15 | 0.02 | 0.09 | 0.03 |
| GI Tract | 6.49 | 0.60 n=4,a | 4.75 | 0.60 | 5.14 | 0.44 | 3.41 | 0.40 |
| Carcass | 47.49 | 1.88 | 45.91 | 5.91 | 45.87 | 3.12 | 31.56 | 4.26 |
| Whole Body Retention | 91.62 | 2.84 | 88.34 | 8.07 | 84.16 | 2.21 | 67.60 | 4.49 |

a - One tissue value rejected due Outlier Statistic >p-95 value.
b - One animal omitted due to tail vein >10 percent
c - One tumor omitted due to weight outside acceptable range.
d - One animal omitted due to 3 or more tissues being outliers.

TABLE VB:Biodistribution of 177Lu-PA-DOTA CC49 Conjugates having a Ratio of 2.02 BFC:Ab, n=4

| Tissue | 5 Hours | | 24 Hours | | 48 Hours | | 120 Hours | |
|---|---|---|---|---|---|---|---|---|
| | Avg. | Std. | Avg. | Std. | Avg. | Std. | Avg. | Std. |
| Blood, Total | 36.53 | 4.47 n=3,a | 24.01 | 3.36 | 20.11 | 3.27 | 12.36 | 1.18 |
| Liver | 10.73 | 1.22 | 8.22 | 0.68 n=4,a | 10.16 | 1.00 | 8.30 | 1.21 |
| Spleen | 0.84 | 0.24 | 0.70 | 0.22 | 0.69 | 0.14 | 0.62 | 0.11 |
| Kidney | 0.97 | 0.06 n-3,a | 1.10 | 0.26 | 0.86 | 0.14 | 0.76 | 0.06 |
| Tumor | 2.52 | 0.47 | 9.57 | 1.09 n=4,a | 11.52 | 1.90 n=4,c | 10.33 | 2.26 |
| Femur | 0.15 | 0.01 | 0.14 | 0.03 | 0.15 | 0.02 | 0.09 | 0.01 |
| GI Tract | 6.56 | 0.30 | 4.85 | 0.66 | 4.48 | 0.38 | 3.21 | 0.36 |
| Carcass | 44.51 | 1.03 n=3,a | 41.26 | 5.43 | 39.93 | 8.49 | 27.84 | 2.23 |
| Whole Body Retention | 88.35 | 5.36 | 83.30 | 4.89 | 76.64 | 10.92 | 59.23 | 3.77 |

a - One tissue value rejected due Outlier Statistic >p-95 value.
b - One animal omitted due to tail vein >10 percent
c - One tumor omitted due to weight outside acceptable range.
d - One animal omitted due to 3 or more tissues being outliers.

Example 13 Preparation of $^{111}In$ PA-DOTA-CC49

To 85 µL (3 x $10^{-9}$ moles) of a chelating conjugate (PA-DOTA-CC49) in 25 mM ammonium acetate buffer at pH7, prepared as described in Example 9, was added 7.5 µL of $^{111}In$ 0.05 N hydrochloric acid, the solution mixed, and allowed to sit at room temperature for 5 minutes. Upon completion of the chelation of the $^{111}In$, 3 µL of a 5 mM DTPA solution was added, and the solution was again mixed. The percent $^{111}In$, associated with the conjugate was determined by HPLC on a gel filtration column (GF-250 column) to be 64 percent. The $^{111}In$ labeled antibody was purified by passing through an anion exchange column (SEPHADEX™ QAE). The labeled antibody was characterized by HPLC, PAGE-SDS gel coupled with autoradiography and immunoreactivity assay. The specific activity of the labeled antibody was estimated to be 1.4 mCi/mg.

When chelation was carried out at 37°C for 15 minutes, 75 percent of $^{111}In$ became antibody bound (see Table VI). The purified $^{111}In$ labeled antibody was identical to that prepared by chelation at room temperature with respect to the immunoreactivity and physical integrity determined by HPLC analysis. The results in Table VI also show that there was little aggregate formation.

TABLE VI:

| Binding of $^{111}In$ to PA-DOTA-CC49 | | |
|---|---|---|
| | chelation at room temp. (percent) | chelation at 37°C (percent) |
| % $^{111}In$ bound to Antibody by HPLC analysis | 99.5 | 99 |
| % aggregates | 0.5% | 0.2% |
| Immunoreactivity by BSM Binding | 99.7±0.01% | 99.8±0.7% |

Example 14 Preparation of $^{153}Sm$ PA-DOTA-CC49

To 85 µL (3 x $10^{-9}$ moles) of a chelating agent conjugate (PA-DOTA-CC49) in 25 mM ammonium actetate buffer at pH 7, prepared as per Example 10, was added 3.8 µL of $^{153}Sm$ with carrier added (2 mM Sm in 0.10 N Hydrochloric acid), the solution was mixed and allowed to sit at room temperature for 15 minutes. Upon completion of the chelation of the $^{153}Sm$, 3 µL of a 5 mM DTPA solution was added, and the solution was again mixed. The percent $^{153}Sm$ associated with the conjugate was determined by HPLC on a gel filtration column (GF-250 column to be 83 ± 12 percent. The $^{153}Sm$ labeled antibody was purified by passing through an anion exchange resin (SEPHADEX™ QAE column), and characterized by HPLC, PAGE-SDS gel coupled with autoradiography and immunoreactivity assay. The results showed that 98.4 percent of the $^{153}Sm$ was bound to antibody with less than 0.3 percent aggregate formation with immunoreactivity by binding to BSM of 97.7 ± 0.35 percent.

Example 15 Preparation of $^{90}Y$ PA-DOTA-CC49

To 85 µL (3 x $10^{-9}$ moles) of a chelating agent conjugate (PA-DOTA-CC49) in 25 mM ammonium acetate buffer at pH 7, prepared as per Example 10, was added 8.3 µL of carrier free $^{90}Y$ in 0.05 N hydrochloric acid, the solution was mixed and allowed to sit at room temperature (about 21 °C) for 30 minutes. Less than 1 percent of $^{90}Y$ was found to be antibody bound. After heating at 37°C for 30 minutes, the yield of $^{90}Y$ bound was found to be 11 percent by HPLC analysis.

In other experiments when cold yttrium actetate was added for 10 minutes, the yield of chelation was greatly improved, these results are shown in Table VII.

TABLE VII:

| Chelation of CC49 PA-DOTA Conjuagte with $^{90}Y$ with various amounts of cold yttrium added, chelation for 10 minutes in 25 mM ammonium acetate, pH7 | | | |
|---|---|---|---|
| run No. | (Yttrium)/-(Antibody) | percent $^{90}Y$ CC49 at | |
| | | room temp (about 21°C) | 37°C |
| 1 | 2.5 | 53.8 | |

TABLE VII: (continued)

| Chelation of CC49 PA-DOTA Conjuagte with $^{90}Y$ with various amounts of cold yttrium added, chelation for 10 minutes in 25 mM ammonium acetate, pH7 | | | |
|---|---|---|---|
| run No. | (Yttrium)/-(Antibody) | percent $^{90}Y$ CC49 at | |
| | | room temp (about 21°C) | 37°C |
| 2 | 2.5 | | 95.9 |
| | | | |
| 3 | 1.25 | 56.1 | |
| 4 | 1.25 | | 95.8 |
| | | | |
| 5 | 0.63 | 61.3 | |
| 6 | 0.63 | | 92.1 |
| | | | |
| 7 | 0.25 | 73.9 | |
| 8 | 0.25 | | 86.7 |
| | | | |
| 9 | 0.05 | 29.1 | |
| 10 | 0.05 | | 50.3 |
| | | | |
| 11 | 0.005 | | 5.0 |
| 12[a] | | | 15.0 |

a. chelation continued for 30 min.

The $^{90}Y$ labeled antibody was purified by passing through a BioRad AG-1 x8 column and characterized by HPLC, and BSM immunoreactivity assay. In general, in the purified material greater than 98 percent of the $^{90}Y$ was bound to the antibody and immunoreactivity, as measured by binding to BMS, was about 93-95 percent.

Example 16 Effect of Various Ions on Chelation

To determine the effect of various ions on chelation of $^{177}Lu$ by SCN-PA-DOTA, in general, 7.5 µL of one of the solutions (0.1M) listed in Table VIII was added to 85 µL (3 nanamoles) of a PA-DOTA-CC49 conjugate in 25 mM ammonium acetate at about pH 7. To this mixture 7.5 µL of a $^{177}Lu$ solution (1 mM in 0.05 N HCl) was added, the solution mixed and then allowed to set for about 10 minutes at room temperature before HPLC analysis. The effect of the various ions on chelation is shown in Table VIII.

TABLE VIII:

| Effect of various ions on chelation of CC49 PA-DOTA Conjugate | |
|---|---|
| Ions or buffers tested | Yield of chelation based on $^{177}Lu$ |
| control (no additives) | 95.8±0.48% |
| sodium acetate | 86.5±5.3% |
| sodium carbonate | 91.8±4.2% |
| sodium chloride | 95.1±0.35% |
| sodium citrate | 0.0% |

TABLE VIII: (continued)

| Effect of various ions on chelation of CC49 PA-DOTA Conjugate | |
|---|---|
| Ions or buffers tested | Yield of chelation based on $^{177}Lu$ |
| sodium phosphate | 0.0% |
| sodium sulfate | 94.5±1.1% |
| calcium chloride | 1.1±0.21% |
| magnesium chloride | 93.7±2.3% |
| manganese chloride | 0.0% |
| potassium chloride | 96.3±0.14% |
| Trisma | 94.9±0.99% |

The results in Table VIII show an inhibitory effect of citrate and phosphate, among the anions; and calcium and manganese among the cations on the chelation of the PA-DOTA-CC49 conjugate.

**Claims**

**1.** A process for preparing a chelate conjugate comprising:

(i) purifying a polyaza chelating agent by chromatography on a silica gel column, wherein the silica gel has been acid washed prior to loading the polyaza chelating agent onto the column and the polyaza chelating agent is of the formula:

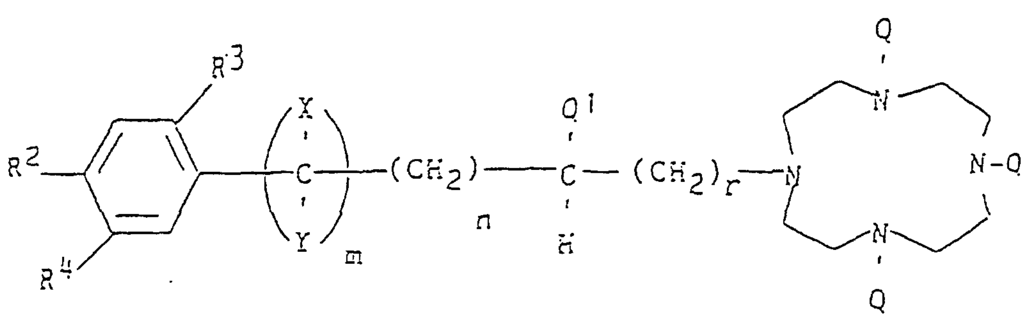

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken with an adjacent X and Y to form an additional carbon-carbon bond;
n is 0 or 1;
m is an integer from 0 to 10 inclusive;
p = 1 or 2;

r = 0 or 1;
w = 0 or 1;

with the proviso that n is only 1 when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
(ii) contacting the purified polyaza chelating agent with thiophosgene in an aqueous environment in the absence of an organic solvent at a pH from about 1 to about 5 to form an isothiocyanato activated polyaza chelating agent and recovering the isothiocyanato activated polyaza chelating agent;
(iii) contacting the isothiocyanato activated polyaza chelating agent with a protein to form a chelating agent conjugate and recovering the chelating agent conjugate;
and
(iv) contacting the chelating agent conjugate with a radionuclide ion to form a chelate conjugate and recovering the chelate conjugate.

**2.** The process of Claim 1 where r is 0 and each Q is $(CHR^5)_pCO_2R$.

**3.** The process of Claim 1 wherein the chelating agent is α-[2-(4-aminophenyl)ethyl]-1,4,7,10-tetraazacyclododecane-1,4,7,10-tetraacetic acid.

**4.** The process of any one of claims 1 through 3 wherein the radionucleus is $^{153}Sm$, $^{166}Ho$, $^{175}Yb$, $^{177}Lu$, $^{159}Gd$, $^{140}La$, $^{142}Pr$, $^{149}Pm$, $^{90}Y$ or $^{111}In$.

**5.** The process of Claim 4 wherein the radionuclide is $^{177}Lu$.

**6.** The process of Claim 1 wherein step (iii) comprises reacting the isothiocyanate activated chelate conjugate with an antibody at about 37°C.

**7.** The process of Claim 1 wherein a chelating agent is added to the chelate conjugate formed in step (iv) to remove excess metal ion.

**8.** A chelate conjugate prepared by the process of Claim 1.

**9.** A process for preparing a chelate conjugate comprising:

(i) purifying a polyaza chelating agent of the formula:

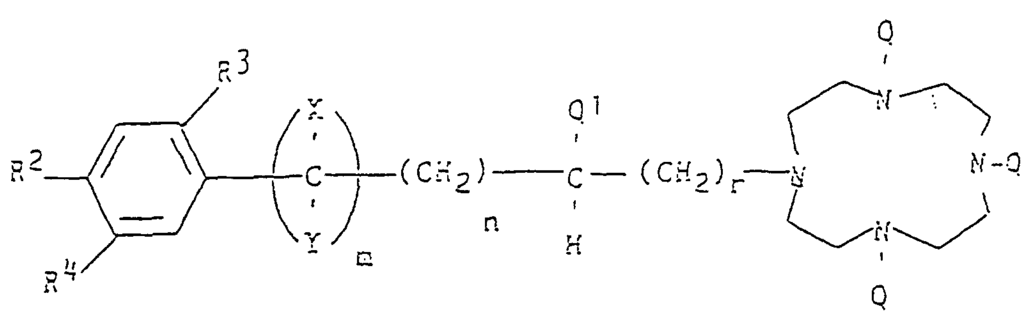

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;

each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is only 1 when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
(ii) contacting the purified polyaza chelating agent with thiophosgene to form an isothiocyanato activated polyaza chelating agent and recovering the isothiocyanato activated polyaza chelating agent;
(iii) contacting the isothiocyanato activated polyaza chelating agent with a protein to form a chelating agent conjugate;
(iv) contacting the chelating agent conjugate with a radionuclide to form a chelate conjugate and recovering the chelate conjugate;

the improvement which comprises:

in step (i): purifying the polyaza chelating agent by chromatography on a silica gel column, wherein the silica gel has been acid washed prior to loading the polyaza chelating agent onto the column;
in step (ii): contacting the purified polyaza chelating agent with thiophosgene in an aqueous environment in the absence of an organic solvent at a pH from about 1 to about 5;
and
in step (iii): contacting the isothiocyanato activated polyaza chelating agent with a biological molecule at between about 25°C to about 40°C.

**10.** The chelate conjugate of Claim 9 wherein the chelate conjugate contains less than about five percent aggregate formation as measured by high performance liquid chromatography.

**11.** A chelating agent conjugate prepared by the process of:

(i) purifying a polyaza chelating agent by chromatography on a silica gel column, wherein the silica gel has been acid washed prior to loading the polyaza chelating agent onto the column and the polyaza chelating agent is of the formula:

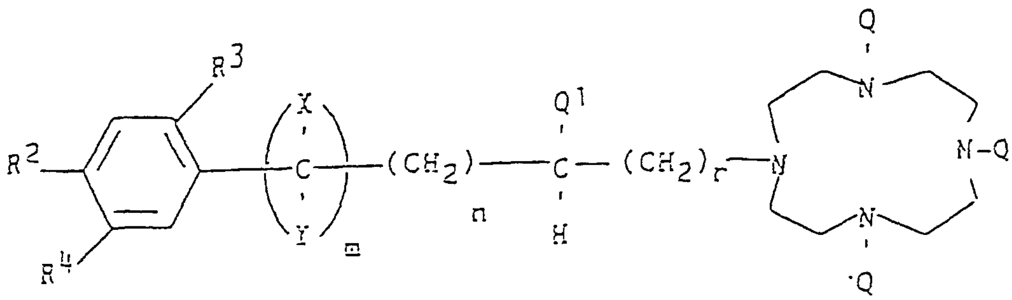

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken independently with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
(ii) contacting the purified polyaza chelating agent with thiophosgene to form an isothiocyanato activated polyaza chelating agent and recovering the isothiocyanato activated polyaza chelating agent; and
(iii) contacting the isothiocyanato activated polyaza chelating agent with a protein to form a chelating agent conjugate.

**12.** The chelating agent conjugate of Claim 11 wherein the chelating agent conjugate contains less than about three percent aggregate formation as measured by high performance liquid chromatography.

**13.** A chelating agent conjugate comprising a protein conjugated to a chelating agent of the formula:

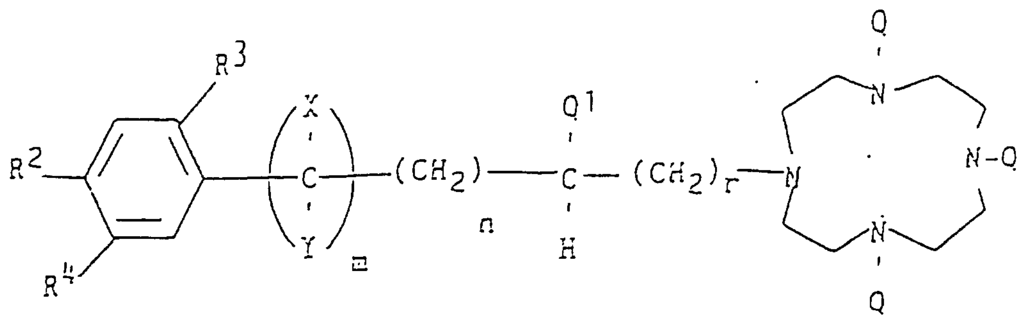

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken independently with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
wherein the chelating agent conjugate, upon conjugation of said chelating agent to said protein, contains less than about three percent aggregate formation as measured by high performance liquid chromatography.

**14.** A chelate conjugate comprising a radionuclide, a chelating agent and a protein wherein the radionuclide is bound by the chelating agent, the chelating agent is conjugated to the protein and the chelating agent is of the formula:

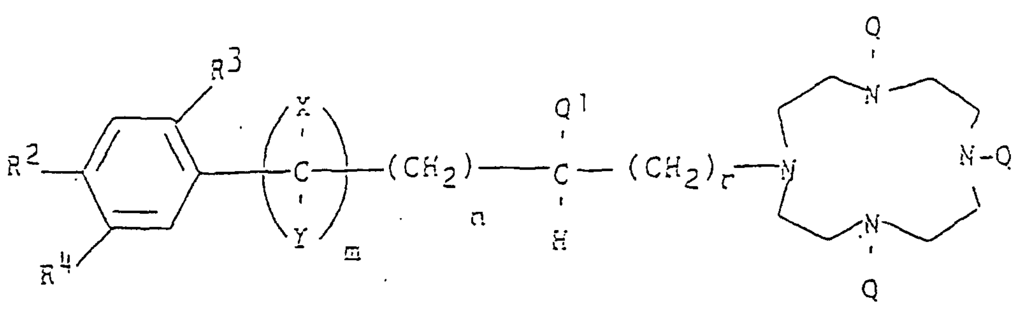

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken independently with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
wherein the chelate conjugate, upon conjugation of said chelate to said protein, contains less than about five percent aggregate formation as measured by high performance liquid chromatography.

**15.** The chelate conjugate of Claim 14 where r is 0 and each Q is $(CHR^5)_pCO_2R$.

**16.** The chelate conjugate of Claim 14 wherein the chelating agent is α-[2-(4-aminophenyl)ethyl]-1,4,7,10-tetraazacyclododecane-1,4,7,10-tetraacetic acid.

**17.** The chelate conjugate of Claim 14 wherein the radionuclide is $^{153}Sm$, $^{166}Ho$, $^{175}Yb$, $^{177}Lu$, $^{159}Gd$, $^{140}La$, $^{142}Pr$, $^{149}Pm$, $^{90}Y$ or $^{111}In$.

**18.** The chelate conjugate of Claim 17 wherein the radionuclide is $^{177}Lu$.

**19.** A process for preparing a chelating agent conjugate comprising:

(i) purifying a polyaza chelating agent by chromatography on a silica gel column, wherein the silica gel has been acid washed prior to loading the polyaza chelating agent onto the column and the polyaza chelating agent is of the formula:

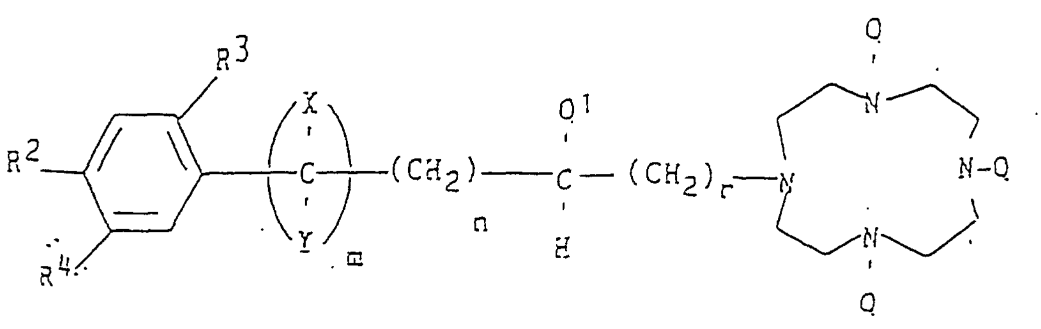

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen, and at least one Q must be $(CHR^5)_pP(O)OHR^6$;

each $R^5$ independently is hydrogen, $C_1$-$C_4$ alkyl or -($C_1$-$C_2$ alkyl)phenyl;
each $R^6$ independently is -OH or $C_1$ - $C_4$ alkyl;
X and Y are each independently hydrogen or may be taken independently with an adjacent X and Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
(ii) contacting the purified polyaza chelating agent with thiophosgene to form an isothiocyanato activated polyaza chelating agent and recovering the isothiocyanato activated polyaza chelating agent; and
(iii) contacting the isothiocyanato activated polyaza chelating agent with a protein to form a chelating agent conjugate.

**20.** A chelating agent conjugate comprising a protein conjugated to a chelating agent of the formula:

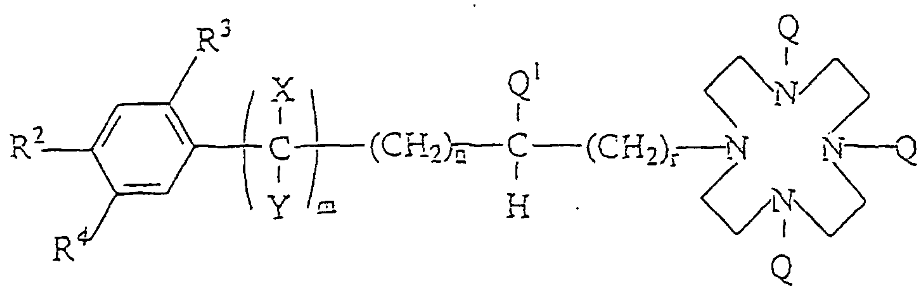

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen;

each $R^5$ independently is hydrogen, $C_1$-$C_4$ alkyl or -($C_1$-$C_2$ alkyl)phenyl;

with the proviso that at least one $R^5$ must be -($C_1$ - $C_2$ alkyl)phenyl;

each $R^6$ independently is -OH or $C_1$ - $C_4$ alkyl;
X and Y are each independently hydrogen or may be taken independently with an adjacent X or Y to form an

additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;
$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
wherein the chelating agent conjugate, upon conjugation of said chelating agent to said protein, contains less than about three percent aggregate formation as measured by high performance liquid chromatography.

**21.** A chelate conjugate comprising a radionuclide, a chelating agent, and a protein wherein the radionuclide is bound by the chelating agent, the chelating agent is conjugated to the protein, and the chelating agent is of the formula:

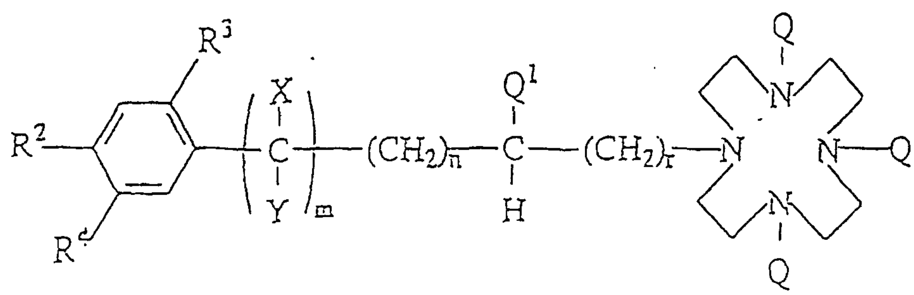

wherein:

each Q is independently hydrogen, $(CHR^5)_pCO_2R$; $(CHR^5)_pPO_3H_2$, or $(CHR^5)_pP(O)OHR^6$;
$Q^1$ is hydrogen or $(CHR^5)_wCO_2R$;
each R independently is hydrogen, benzyl or $C_1$-$C_4$ alkyl;

with the proviso that at least two of the sum of Q and $Q^1$ must be other than hydrogen;

each $R^5$ independently is hydrogen, $C_1$-$C_4$ alkyl or -($C_1$-$C_2$ alkyl)phenyl;

with the proviso that at least one $R^5$ must be -($C_1$ - $C_2$ alkyl)phenyl;

each $R^6$ independently is -OH or $C_1$ - $C_4$ alkyl;
X and Y are each independently hydrogen or may be taken independently with an adjacent X or Y to form an additional carbon-carbon bond;
n is 0 or 1;
m is an integer from 0 to 10 inclusive;
p = 1 or 2;
r = 0 or 1;
w = 0 or 1;

with the proviso that n is 1 only when X and/or Y form an additional carbon-carbon bond and the sum of r and w is 0 or 1;

$R^2$ and $R^4$ are independently hydrogen or amino;

$R^3$ is selected from the group consisting of $C_1$-$C_4$ alkoxy, - $OCH_2CO_2H$, hydroxy and hydrogen;

with the proviso that $R^2$ and $R^4$ cannot both be hydrogen but one of $R^2$ and $R^4$ must be hydrogen;
wherein the chelate conjugate, upon conjugation of said chelate to said protein, contains less than about three percent aggregate formation as measured by high performance liquid chromatography.

**Patentansprüche**

**1.** Verfahren zur Herstellung eines Chelat-Konjugats umfassend:

(i) reinigen eines Polyaza-Chelatierungsmittels durch Chromatographie an einer Kieselgelsäule, wobei das Kieselgel vor dem Laden des Polyaza-Chelatierungsmittels auf die Säule mit Säure gewaschen wurde und das Polyaza-Chelatierungsmittel die Formel:

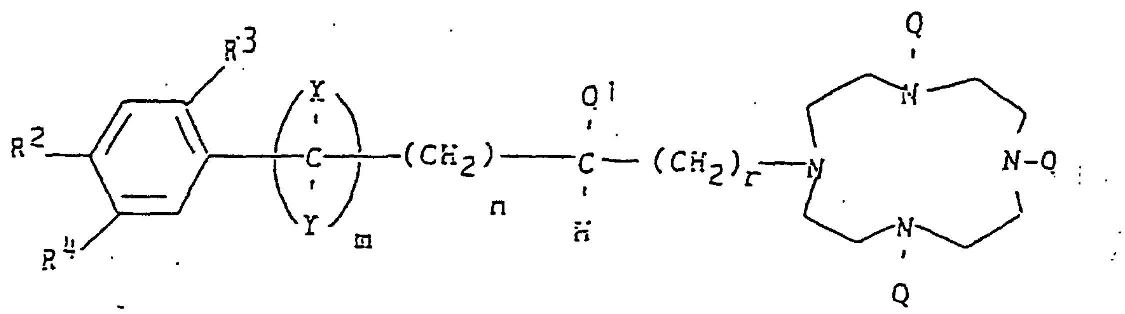

besitzt, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder mit einem benachbarten
X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, $-OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
(ii) in Kontakt bringen des gereinigten Polyaza-Chelatierungsmittels mit Thiophosgen in einer wässrigen Umgebung in Abwesenheit eines organischen Lösungsmittels bei einem pH-Wert von ungefähr 1 bis ungefähr 5 um ein Isothiocyanat aktiviertes Polyaza-Chelatierungs-Mittel zu bilden und Gewinnen des Isothiocyanat aktivierten Polyaza-Chelatierungsmittels;

(iii) in Kontakt bringen des Isothiocyanat aktivierten Polyaza-Chelatierungsmittels mit einem Protein um ein Chelatierungsmittel-Konjugat zu bilden und Gewinnen des Chelatierungsmittel-Konjugats;
und
(iv) in Kontakt bringen des Chelatierungsmittel-Konjugats mit einem Radionuclid-Ion um ein Chelat-Konjugat zu bilden und Gewinnen des Chelat-Konjugats.

**2.** Verfahren nach Anspruch 1, wobei r 0 ist und jedes Q $(CHR^5)_pCO_2R$ ist.

**3.** Verfahren nach Anspruch 1, wobei das Chelatierungsmittel α-[2-(4-Aminophenyl) ethyl]-1,4,7,10-tetraazacyclododekan-1,4,7,10-tetraessigsäure ist.

**4.** Verfahren nach einem der Ansprüche 1 bis 3 wobei das Radionuclid $^{153}Sm$, $^{166}Ho$, $^{175}Yb$, $^{177}Lu$, $^{159}Gd$, $^{140}La$, $^{142}Pr$, $^{149}Pm$, $^{90}Y$ oder $^{111}In$ ist.

**5.** Verfahren nach Anspruch 4, wobei das Radionuclid $^{177}Lu$ ist.

**6.** Verfahren nach Anspruch 1, wobei Schritt (iii) Umsetzen des Isothiocyanat-aktivierten Chelatkonjugats mit einem Antikörper bei ungefähr 37°C umfasst.

**7.** Verfahren nach Anspruch 1, wobei ein Chelatierungsmittel zu dem in Schritt (iv) gebildeten Chelatkonjugat zugegeben wird, um überschüssiges Metallion zu entfernen.

**8.** Chelatkonjugat welches mit dem Verfahren von Anspruch 1 hergestellt ist.

**9.** Verfahren zur Herstellung eines Chelatkonjugats umfassend:

(i) reinigen eines Polyaza-Chelatierungsmittels der Formel:

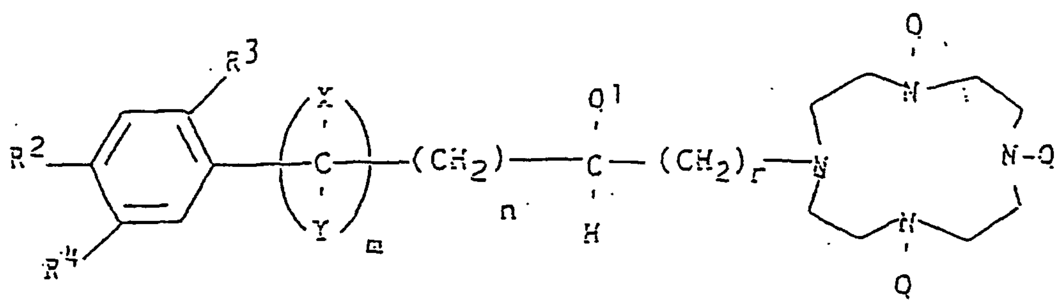

wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder mit einem benachbarten
X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;

w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, $-OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
(ii) in Kontakt bringen des gereinigten Polyaza-Chelatierungsmittels mit Thiophosgen um ein Isothiocyanat aktiviertes Polyaza-Chelatierungsmittel zu bilden und Gewinnen des Isothiocyanat aktivierten Polyaza-Chelatierungsmittels;
(iii) in Kontakt bringen des Isothiocyanat aktivierten Polyaza-Chelatierungsmittels mit einem Protein um ein Chelatierungsmittel-Konjugat zu bilden;
(iv) in Kontakt bringen des Chelatierungsmittelkonjugats mit einem Radionuclid um ein Chelatkonjugat zu bilden und Gewinnen des Chelatkonjugats;

wobei die Verbesserung umfasst:

in Schritt (i): Reinigen des Polyaza-Chelatierungsmittels durch Chromatographie an einer Kieselgelsäule, wobei das Kieselgel vor dem Laden des Polyaza-Chelatierungsmittels auf die Säule mit Säure gewaschen wurde;
in Schritt (ii): in Kontakt bringen des gereinigten Polyaza-Chelatierungsmittels mit Thiophosgen in einer wässrigen Umgebung in Abwesenheit eines organischen Lösungsmittels bei einem pH-Wert von ungefähr 1 bis ungefähr 5;
und
in Schritt (iii): in Kontakt bringen des Isothiocyanat-aktivierten Polyaza-Chelatierungsmittels mit einem biologischen Molekül bei zwischen ungefähr 25°C und ungefähr 40 °C.

**10.** Chelatkonjugat nach Anspruch 9 wobei das Chelatkonjugat weniger als ungefähr fünf Prozent Aggregatbildung enthält, wie durch Hochleistungs-Flüssigkeitschromatographie gemessen.

**11.** Chelatierungsmittelkonjugat hergestellt durch das Verfahren von:

(i) Reinigen eines Polyaza-Chelatierungsmittels durch Chromatographie an einer Kieselgelsäule, wobei das Kieselgel vor dem Laden des Polyaza-Chelatierungsmittels auf die Säule mit Säure gewaschen wurde und das Polyaza-Chelatierungsmittel die Formel:

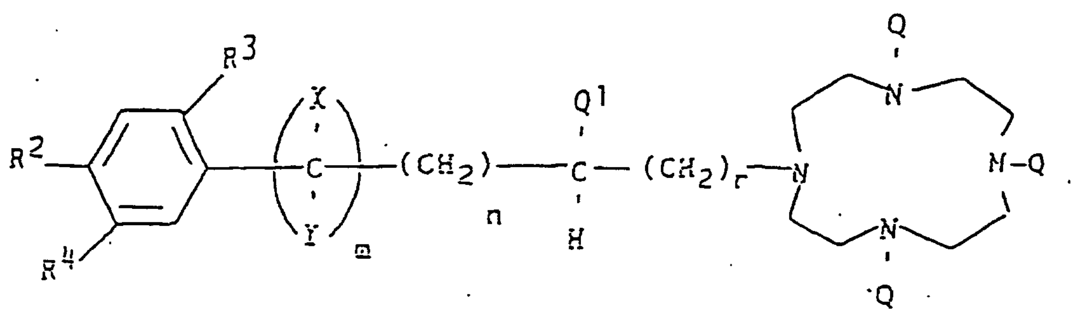

aufweist, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, -$OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
(ii) in Kontakt bringen des gereinigten Polyaza-Chelatierungsmittels mit Thiophosgen um ein Isothiocyanat-aktiviertes Polyaza-Chelatierungsmittel zu bilden und Gewinnen des Isothiocyanat aktivierten Polyaza-Chelatierungsmittels; und
(iii) in Kontakt bringen des Isothiocyanat-aktivierten Polyaza-Chelatierungsmittels mit einem Protein um ein Chelatierungsmittel-Konjugat zu bilden.

**12.** Chelatierungsmittelkonjugat nach Anspruch 11, wobei das Chelatierungsmittelkonjugat weniger als ungefähr drei Prozent Aggregatbildung enthält wie durch Hochleistungs-Flüssigkeitschromatographie gemessen.

**13.** Chelatierungsmittelkonjugat umfassend ein Protein das an ein Chelatierungsmittel der Formel:

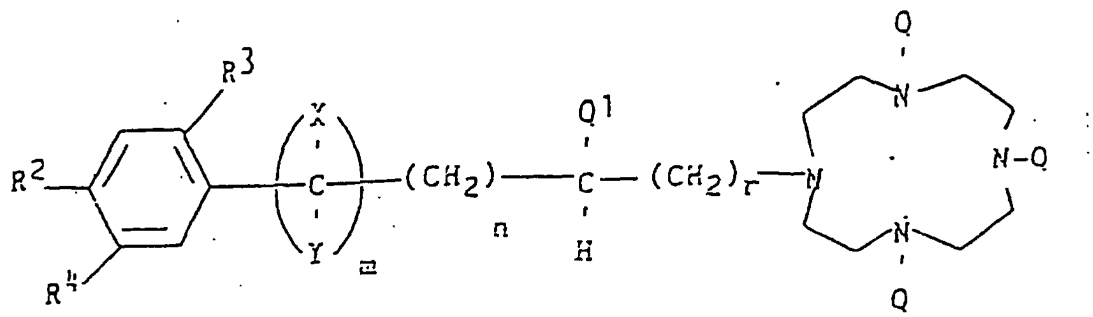

konjugiert ist, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;

r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, -$OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
wobei das Chelatierungsmittelkonjugat, nach Konjugation des Chelatierungsmittels an das Protein weniger als ungefähr drei Prozent Aggregatbildung enthält, wie durch Hochleistungs-Flüssigkeitschromatographie gemessen.

**14.** Chelatkonjugat umfassend ein Radionuclid, ein Chelatierungsmittel und ein Protein, wobei das Radionuclid durch das Chelatierungsmittel gebunden ist, das Chelatierungsmittel an das Protein konjugiert ist und das Chelatierungsmittel die Formel:

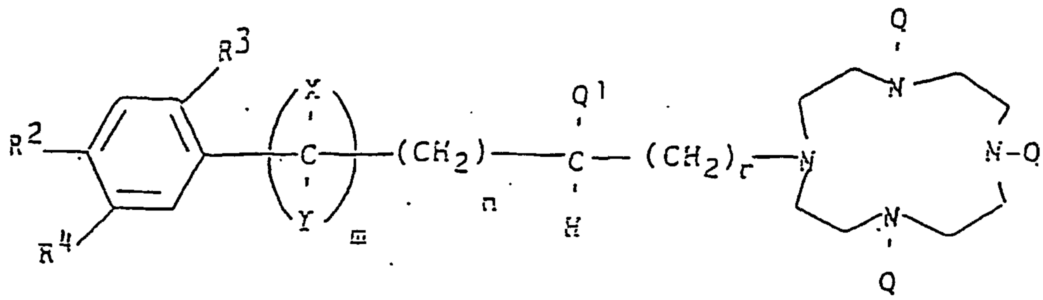

aufweist, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, -$OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
wobei das Chelatkonjugat, nach Konjugation des Chelatierungsmittels an das Protein weniger als ungefähr fünf

Prozent Aggregatbildung enthält, wie durch Hochleistungs-Flüssigkeitschromatographie gemessen.

**15.** Chelatkonjugat nach Anspruch 14, wobei r 0 ist und jedes Q $(CHR^5)_pCO_2R$ ist.

**16.** Chelatkonjugat nach Anspruch 14, wobei das Chelatierungsmittel α-[2-(4-Aminophenyl)ethyl]-1,4,7,10-tetraazacyclododekan-1,4,7,10-tetraessigsäure ist.

**17.** Chelatkonjugat nach Anspruch 14 wobei das Radionuclid $^{153}Sm$, $^{166}Ho$, $^{175}Yb$, $^{177}Lu$, $^{159}Gd$, $^{140}La$, $^{142}Pr$, $^{149}Pm$, $^{90}Y$ oder $^{111}In$ ist.

**18.** Chelatkonjugat nach Anspruch 17, wobei das Radionuclid $^{177}Lu$ ist.

**19.** Verfahren zur Herstellung eines Chelatierungsmittelkonjugats umfassend:

(i) Reinigen eines Polyaza-Chelatierungsmittels durch Chromatographie an einer Kieselgelsäule, wobei das Kieselgel vor dem Laden des Polyaza-Chelatierungsmittels auf die Säule mit Säure gewaschen wurde und das Polyaza-Chelatierungsmittel die Formel:

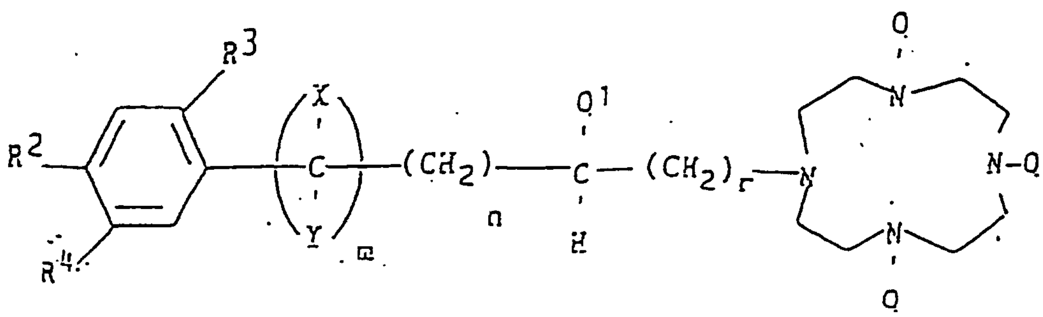

besitzt, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q^1$ von Wasserstoff verschieden sein müssen und mindestens ein Q $(CHR^5)_pP(O)OHR^6$ sein muss;

jedes $R^5$ unabhängig Wasserstoff, $C_1$-$C_4$ Alkyl oder -($C_1$-$C_2$ Alkyl)phenyl ist;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, $-OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
(ii) in Kontakt bringen des gereinigten Polyaza-Chelatierungsmittels mit Thiophosgen um ein Isothiocyanat

aktiviertes Polyaza-Chelatierungsmittel zu bilden und Gewinnen des Isothiocyanat aktivierten Polyaza-Chelatierungsmittels;

(iii) in Kontakt bringen des Isothiocyanat aktivierten Polyaza-Chelatierungsmittels mit einem Protein um ein Chelatierungsmittel-Konjugat zu bilden.

**20.** Chelatierungsmittelkonjugat umfassend ein Protein das an ein Chelatierungsmittel der Formel:

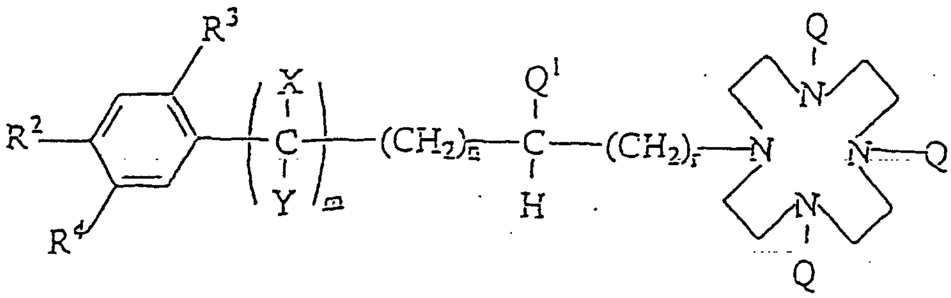

konjugiert ist, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist;

mit der Bedingung, dass mindestens zwei von allen Q und $Q_1$ von Wasserstoff verschieden sein müssen;

jedes $R^5$ unabhängig Wasserstoff, $C_1$-$C_4$ Alkyl oder -($C_1$-$C_2$ Alkyl)phenyl ist; mit der Bedingung, dass mindestens ein $R^5$ -($C_1$-$C_2$ Alkyl)phenyl sein muss;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, - $OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
wobei das Chelatierungsmittelkonjugat nach Konjugation des Chelatierungsmittels an das Protein weniger als ungefähr drei Prozent Aggregatbildung enthält wie durch Hochleistungs-Flüssigkeitschromatographie gemessen.

**21.** Chelatkonjugat umfassend ein Radionuclid, ein Chelatierungsmittel und ein Protein, wobei das Radionuclid durch das Chelatierungsmittel gebunden ist, das Chelatierungsmittel an das Protein konjugiert ist und das Chelatierungsmittel die Formel:

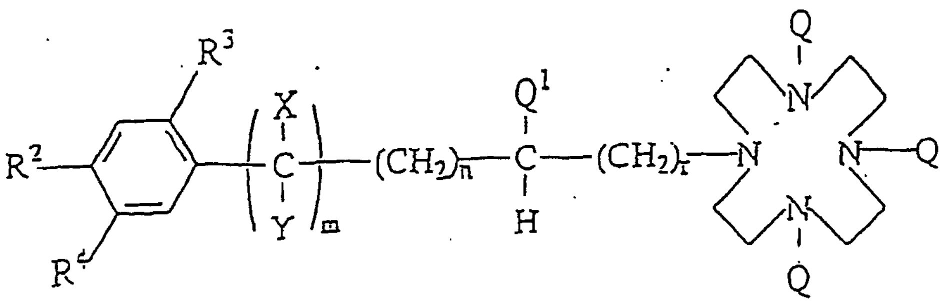

aufweist, wobei:

jedes Q unabhängig Wasserstoff, $(CHR^5)_pCO_2R$; $(CHR^5)_pPO_3H_2$ oder $(CHR^5)_pP(O)OHR^6$ ist;
$Q^1$ Wasserstoff oder $(CHR^5)_wCO_2R$ ist;
jedes R unabhängig Wasserstoff, Benzyl oder $C_1$-$C_4$ Alkyl ist; mit der Bedingung, dass mindestens zwei von der Summe von Q und $Q^1$ von Wasserstoff verschieden sein müssen;
jedes $R^5$ unabhängig Wasserstoff, $C_1$-$C_4$ Alkyl oder -($C_1$-$C_2$ Alkyl)phenyl ist; mit der Bedingung, dass mindestens ein $R^5$ -($C_1$-$C_2$ Alkyl)phenyl sein muss;
jedes $R^6$ unabhängig -OH oder $C_1$-$C_4$ Alkyl ist;
X und Y jeweils unabhängig Wasserstoff sind oder unabhängig mit einem benachbarten X und Y verwendet werden können um eine zusätzliche Kohlenstoff-Kohlenstoff Bindung zu bilden;
n 0 oder 1 ist;
m eine ganze Zahl von 0 bis einschließlich 10 ist;
p = 1 oder 2;
r = 0 oder 1;
w = 0 oder 1;

mit der Bedingung, dass n nur dann 1 ist, wenn X und/oder Y eine zusätzliche Kohlenstoff-Kohlenstoff Bindung bilden und die Summe von r und w 0 oder 1 ist;

$R^2$ und $R^4$ unabhängig Wasserstoff oder Amino sind;
$R^3$ ausgewählt wird aus der Gruppe bestehend aus $C_1$-$C_4$ Alkoxy, - $OCH_2CO_2H$, Hydroxy und Wasserstoff;

mit der Bedingung, dass $R^2$ und $R^4$ nicht beide Wasserstoff sein können aber eines von $R^2$ und $R^4$ Wasserstoff sein muss;
wobei das Chelatkonjugat, bei Konjugation des Chelats an das Protein weniger als ungefähr drei Prozent Aggregatbildung enthält wie durch Hochleistungs-Flüssigkeitschromatographie gemessen.

## Revendications

**1.** Procédé pour préparer un conjugé de chélate, qui comprend :

(i) la purification d'un agent chélateur polyaza par chromatographie sur une colonne de gel de silice, le gel de silice étant lavé avec un acide avant l'introduction de l'agent chélateur polyaza sur la colonne et l'agent chélateur polyaza répondant à la formule :

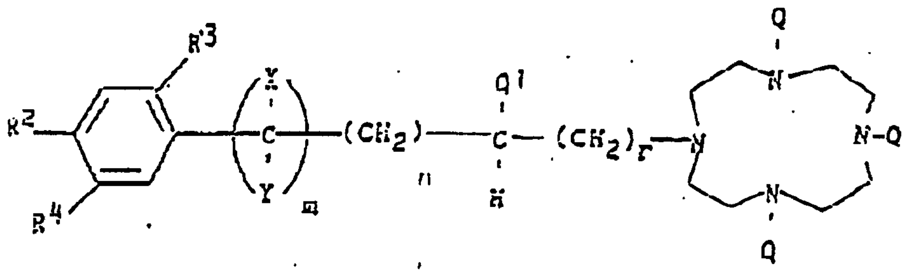

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ;
(ii) la mise en contact de l'agent chélateur polyaza purifié avec du thiophosgène dans un milieu aqueux, en l'absence de solvant organique, à un pH d'environ 1 à environ 5, pour former un agent chélateur polyaza activé par un groupe isothiocyanato, et la récupération de l'agent chélateur polyaza activé par un groupe isothiocyanato ;
(iii) la mise en contact de l'agent chélateur polyaza activé par un groupe isothiocyanato avec une protéine pour former un conjugué de l'agent chélateur, et la récupération du conjugué de l'agent chélateur ; et
(iv) la mise en contact du conjugué de l'agent chélateur avec un ion de radionucléide pour former un conjugué de chélate, et la récupération du conjugué de chélate.

**2.** Procédé selon la revendication 1, dans lequel r est égal à 0 et chaque Q représente un groupe $(CHR^5)_pCO_2R$.

**3.** Procédé selon la revendication 1. dans lequel l'agent chélateur est l'acide α-[2-(4-aminophényl)éthyl]-1,4,7,10-tétraazacyclododécane-1,4,7,10-tétraacétique.

**4.** Procédé selon l'une quelconque des revendications 1 à 3, dans lequel le radionucléide est $^{153}Sm$, $^{166}HO$, $^{175}Yb$, $^{177}Lu$, $^{159}Gd$, $^{140}La$, $^{142}Pr$, $^{149}Pm$, $^{90}Y$ ou $^{111}In$.

**5.** Procédé selon la revendication 4, dans lequel le radionucléide est $^{177}Lu$.

**6.** Procédé selon la revendication 1, dans lequel l'étape (iii) comprend la réaction de l'agent chélateur polyaza activé par un groupe isothiocyanato avec un anticorps à environ 37°C.

**7.** Procédé selon la revendication 1, dans lequel un ajoute un agent chélateur au conjugué de chélate formé dans l'étape (iv) pour éliminer l'ion métallique en excès.

**8.** Conjugué de chélate préparé par le procédé selon la revendication 1.

**9.** Procédé pour préparer un conjugué de chélate, qui comprend :

(i) la purification d'un agent chélateur polyaza de formule :

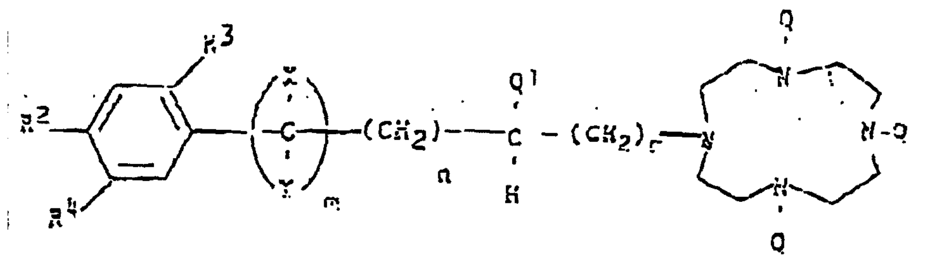

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;
étant entendu que $R^2$ et $R^4$ ne peuvent pas être simultanément des atomes d'hydrogène, mais que l'un de $R^2$ et $R^4$ doit être un atome d'hydrogène ;

(ii) la mise en contact de l'agent chélateur polyaza purifié avec du thiophosgène pour former un agent chélateur polyaza activé par un groupe isothiocyanato, et la récupération de l'agent chélateur polyaza activé par un groupe isothiocyanato ;
(iii) la mise en contact de l'agent chélateur polyaza activé par un groupe isothiocyanato avec une protéine,

pour former un conjugué d'agent chélateur ; et
(iv) la mise en contact du conjugué d'agent chélateur avec un radionucléide pour former un conjugué de chélate, et la récupération du conjugné de chélate ;

ledit procédé comprenant les améliorations suivantes :

dans l'étape (i) : la purification de l'agent chélateur polyaza par chromatographie sur une colonne de gel de silice, le gel de silice étant lavé par un acide avant l'introduction de l'agent chélateur polyaza sur la colonne ;
dans l'étape (ii) : la mise en contact de l'agent chélateur polyaza purifié avec du thiophosgène dans un milieu aqueux, en l'absence de solvant organique, à un pH d'environ 1 à environ 5 ;
et
dans l'étape (iii) : la mise en contact de l'agent chélateur polyaza activé par un groupe isothiocyanato avec une molécule biologique à une température comprise entre environ 25°C et environ 40°C.

**10.** Conjugué de chélate selon la revendication 9, qui contient moins d'environ 5% de formation d'agrégat, ce pourcentage étant mesuré par chromatographie en phase liquide à haute performance.

**11.** Conjugué d'agent chélateur qui est préparé par le procédé consistant à :

(i) purifier un agent chélateur polyaza par chromatographie sur une colonne de gel de silice, le gel de silice étant lavé par un acide avant l'introduction de l'agent chélateur polyaza sur la colonne, et l'agent chélateur polyaza répondant à la fomule :

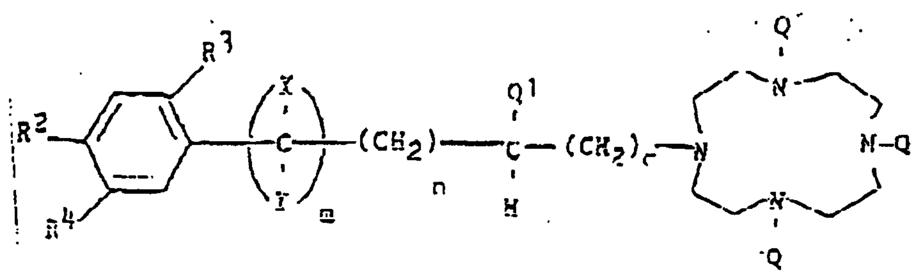

dans laquelle ;

chaque Q représente, indépendamment, un atome d'hydrogène ou un groupe $(CHR^5)_pCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$ ;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et

que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ;
(ii) mettre en contact l'agent chélateur polyaza purifié avec du thiophosgène pour former un agent chélateur polyaza activé par un groupe isothiocyanato, et récupérer l'agent chélateur polyaza activé par un groupe isothiocyanato ; et
(iii) mettre en contact l'agent chélateur pulyaza activé par un groupe isothiocyanato avec une protéine, pour former un conjugué d'agent chélateur.

**12.** Conjugué d'agent chélateur selon la revendication 11, qui contient moins d'environ 3% de formation d'agrégat, ce pourcentage étant mesuré par chromatographie en phase liquide à haute performance.

**13.** Conjugué d'agent chélateur, qui comprend une protéine associée à un agent chélateur de formule :

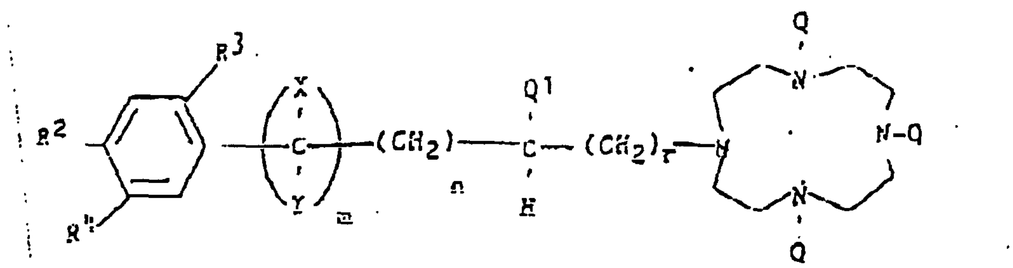

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$;

étant entendu qu'au moins deux des Q et $Q^1$ doivent être autres que des atomes d'hydrogène et qu'au moins un Q doit être un groupe $(CHR^5)_pP(O)OHR^6$;

chaque $R^5$ représente, indépendamment, un atome d'hydrogène, un groupe alkyle en $C_1$ à $C_4$ ou un groupe (alkyl en $C_1$ à $C_2$)phényle ;
chaque R" représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone carbone supplémentaire ;
n est égal à 0 ou 1 ;
m désigne un nombre entier ayant une valeur de 0 à 10, bornes incluses ;
p est égal à 1 on 2 ;
r est égal à 0 ou 1 ;
w est égal à 0 ou 1 ;

érant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ;
ledit conjugué d'agent chélateur, obtenu par union dudit agent chélateur avec ladite protéine, contenant moins d'environ 3% de formation d'agrégat, ce pourcentage étant mesuré par chromatographie en phase liquide à haute performance.

**14.** Conjugué de chélate comprenant un radionucléide, un agent chélateur et une protéine, dans lequel le radionucléide est fixé par l'agent chélateur, l'agent chélateur est associé à la protéine et l'agent chélateur est un composé de formule :

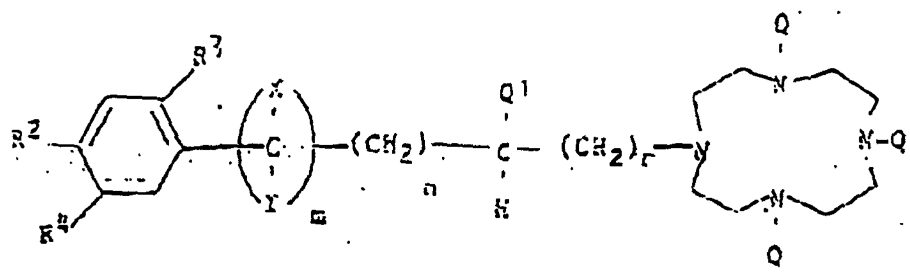

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$ ;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ; ledit conjugué de chélate, obtenu par union dudit chélate avec ladite protéine, contenant moins d'environ 5% de formation d'agrégat, ce pourcentage étant mesuré par chromatographie en phase liquide à haute performance.

**15.** Conjugué de chélate selon la revendication 14, dans lequel r est égal à 0 et chaque Q représente un groupe $(CHR^5)_pCO_2R$,

**16.** Conjugué de chélate selon la revendication 14, dans lequel l'agent chélateur est l'acide α-[2-(4-aminophényl)éthyl]-1,4,7,10-tétraazacyclododécane-1,4,7,10-tétracétique.

**17.** Conjugué de chélate selon la revendication 14, dans lequel le radionucléide est $^{153}Sm$, $^{166}HO$, $^{175}Yb$, $^{177}Lu$, $^{159}Gd$, $^{140}La$, $^{142}Pr$, $^{149}Pm$, $^{90}Y$ ou $^{111}In$.

**18.** Coujugué de chélate selon la revendication 17, dans lequel le radionucléide est $^{177}Lu$.

**19.** Procédé pour préparer un conjugué d'agent chélateur, qui comprend :

(i) la purification d'un agent chélateur polyaza par chromatographie sur une colonne de gel de silice, le gel de silice étant lavé avec un acide avant l'introduction de l'agent chélateur polyaza sur la colonne et l'agent chélateur polyaza répondant à la formule :

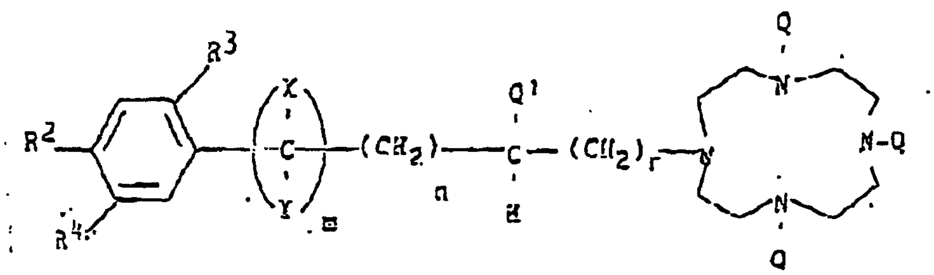

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$ ;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être ou atome d'hydrogène ;
(ii) la mise en contact de l'agent chélateur polyaza purifié avec du thiophosgène pour former un agent chélateur polyaza activé par un groupe isothiocyanato, et la récupération de l'agent chélateur polyaza activé par un groupe isothiocyanato ; et

(iii) la mise en contact de l'agent chélateur polyaza activé par un groupe isothiocyanato avec une protéine pour former un conjugué d'agent chélateur.

**20.** Conjugué d'agent chélateur, qui comprend une protéine associée à un agent chélateur de formule :

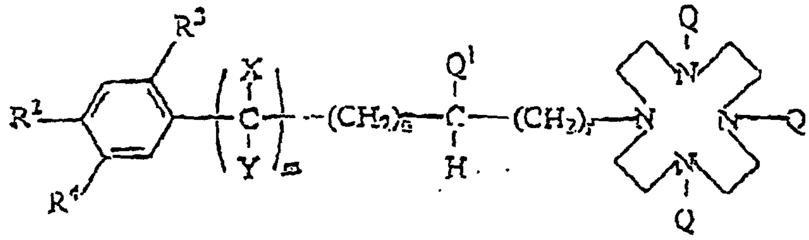

dans laquelle :

chaque Q représente, indépendamment, un atome d'hydrogène ou un groupe $(CHR^5)_pCO_2R$, $(CHR^5)_pPO_3H_2$, ou $(CHR^5)_pP(O)OHR^6$;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$ ;

chaque $R^5$ représente, indépendamment, un atome d'hydrogène, un groupe alkyle en $C_1$ à $C_4$ ou un groupe (alkyl en $C_1$ à $C_2$)phényle ;
chaque $R^6$ représente, indépendamment, un groupe -OH ou alkyle en $C_1$ à $C_4$ ;
X at Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en C, à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ;
ledit conjugué d'agent chélateur, obtenu par union dudit agent chélateur avec ladite protéine, contenant moins d'environ 3% de formation d'agrégat, ce pourcentage étant mesuré par chromatographie en phase liquide à haute performance.

**21.** Conjugué de chélate comprenant un radionucléide, un agent chélateur et une protéine, dans lequel le radionucléide est fixé par l'agent chélateur, l'agent chélateur est associé à la protéine, et l'agent chélateur répond à la formule :

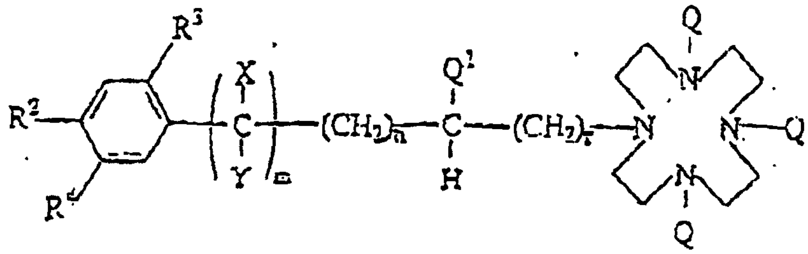

dans laquelle :

chaque Q représente, indépendamment, un atome d'hydrogène ou un groupe $(CHR^5)_wCO_2R$, $(CHR^5)_pPO_3H_2$, ou $(CHR^5)_pP(O)OHR^6$ ;
$Q^1$ représente un atome d'hydrogène ou un groupe $(CHR^5)_wCO_2R$ ;
chaque R représente, indépendamment, un atome d'hydrogène, un groupe benzyle ou un groupe alkyle en $C_1$ à $C_4$ ;

étant entendu qu'au moins deux des Q et $Q^1$ doivent être autres que des atomes d'hydrogène et qu'au moins un Q doit être un groupe $(CHR^5)_pP(O)OHR^6$ ;

chaque $R^5$ représente, indépendamment, un atome d'hydrogène, un groupe alkyle en $C_1$ à $C_4$ on un groupe (alkyl en $C_1$ à $C_2$)phényle ;
chaque $R^6$ représente, indépendamment, un groupe -OH ou alkyle en $C_1$ à $C_4$ ;
X et Y représentent chacun, indépendamment, un atome d'hydrogène ou peuvent être pris avec un X ou Y adjacent pour former une liaison carbone-carbone supplémentaire ;
n est égal à 0 ou 1 ;
m désigne un nombre entier ayant une valeur de 0 à 10, bornes incluses ;
p est égal à 1 ou 2 ;
r est égal à 0 ou 1 ;
w est égal à 0 ou 1 ;

étant entendu que n est égal à 1 lorsque X et/ou Y forment une liaison carbone-carbone supplémentaire et que la somme de r et w est égale à 0 ou 1 ;

$R^2$ et $R^4$ représentent chacun, indépendamment, un atome d'hydrogène ou un groupe amino ;
$R^3$ représente un atome d'hydrogène ou un groupe alcoxy en $C_1$ à $C_4$, $-OCH_2CO_2H$ ou hydroxyle ;

étant entendu que $R^2$ et $R^4$ ne peuvent pas être simultanément des atomes d'hydrogène, mais que l'un de $R^2$ et $R^4$ doit être un atome d'hydrogène ; ledit conjugué de chélate, obtenu par union dudit chélate avec ladite protéine, contenant moins d'environ 3% de formation d'agrégat, ce pourcentage étant mesuré par chromatographie en phase liquide à haute performance.